# EUROPEAN PATENT SPECIFICATION

(11) **EP 2 313 429 B1**
(45) Date of publication and mention of the grant of the patent: **10.09.2014**
(21) Application number: 09772185.6
(22) Date of filing: 03.07.2009
(51) Int. Cl.: C07K 14/445, C12N 15/67, A61K 39/015, A61K 39/00

(54) **MSP-1 protein preparations from plasmodium**
MSP-1-Proteinzubereitungen aus Plasmodium
Préparation de protéine MSP-1 à partir de Plasmodium

(30) Priority: 04.07.2008 EP 08012149
(43) Date of publication of application: 27.04.2011
(73) Proprietor: Ruprecht-Karls-Universität Heidelberg, 69117 Heidelberg (DE)
(72) Inventor: LUTZ, Rolf, 76761 Rülzheim (DE); EPP, Christian, 69124 Heidelberg (DE); WÖHLBIER, Ute, Seattle WA 98103 (US); KAUTH, Christian, 69115 Heidelberg (DE)
(74) Representative: Müller-Boré & Partner Patentanwälte PartG mbB
(86) International application number: PCT/EP2009/004826
(87) International publication number: WO 2010/000485

(56) References cited:
- EP-A- 1 637 602
- EP-A- 1 944 313
- WO-A-98/14583
- WO-A-2004/038024
- US-A1- 2002 144 299
- KAUTH CHRISTIAN W ET AL: "The merozoite surface protein 1 complex of human malaria parasite Plasmodium falciparum: interactions and arrangements of subunits" JOURNAL OF BIOLOGICAL CHEMISTRY, AMERICAN SOCIETY OF BIOLOCHEMICAL BIOLOGISTS, BIRMINGHAM,; US, vol. 278, no. 25, 20 June 2003 (2003-06-20), pages 22257-22264, XP002432919 ISSN: 0021-9258 cited in the application
- WOEHLBIER UTE ET AL: "Analysis of antibodies directed against merozoite surface protein 1 of the human malaria parasite Plasmodium falciparum" INFECTION AND IMMUNITY, AMERICAN SOCIETY FOR MICROBIOLOGY. WASHINGTON, vol. 74, no. 2, 1 February 2006 (2006-02-01), pages 1313-1322, XP002432920 ISSN: 0019-9567

### SEQUENCE LISTING

<110> Bujard, Hermann
<120> MSP-1 Protein Preparation from Plasmodium
<130> PCT56225FZ154pau
<140> not yet assigned
   <141> 2009-07-03
<150> EP 08 012 149.4
   <151> 2008-07-04
<160> 10
<170> PatentIn version 3.3
<210> 1
   <211> 893
   <212> PRT
   <213> Artificial sequence
<220>
   <223> p83/30 from P. falciparum strain 3D7
<400> 1
   Met Val Thr His Glu Ser Tyr Gln Glu Leu Val Lys Lys Leu Glu Ala
   1               5                   10                  15
   Leu Glu Asp Ala Val Leu Thr Gly Tyr Ser Leu Phe Gln Lys Glu Lys
               20                  25                  30
   Met Val Leu Asn Glu Glu Glu Ile Thr Thr Lys Gly Ala Ser Ala Gln
           35                  40                  45
   Ser Gly Ala Ser Ala Gln Ser Gly Ala Ser Ala Gln Ser Gly Ala Ser
       50                  55                  60
   Ala Gln Ser Gly Ala Ser Ala Gln Ser Gly Ala Ser Ala Gln Ser Gly
   65                  70                  75                  80
   Thr Ser Gly Pro Ser Gly Pro Ser Gly Thr Ser Pro Ser Ser Arg Ser
                   85                  90                  95
   Asn Thr Leu Pro Arg Ser Asn Thr Ser Ser Gly Ala Ser Pro Pro Ala
               100                 105                 110
   Asp Ala Ser Asp Ser Asp Ala Lys Ser Tyr Ala Asp Leu Lys His Arg
           115                 120                 125
   Val Arg Asn Tyr Leu Phe Thr Ile Lys Glu Leu Lys Tyr Pro Glu Leu
       130                 135                 140
   Phe Asp Leu Thr Asn His Met Leu Thr Leu Cys Asp Asn Ile His Gly
   145                 150                 155                 160
   Phe Lys Tyr Leu Ile Asp Gly Tyr Glu Glu Ile Asn Glu Leu Leu Tyr
                   165                 170                 175
   Lys Leu Asn Phe Tyr Phe Asp Leu Leu Arg Ala Lys Leu Asn Asp Val
               180                 185                 190
   Cys Ala Asn Asp Tyr Cys Gln Ile Pro Phe Asn Leu Lys Ile Arg Ala
           195                 200                 205
   Asn Glu Leu Asp Val Leu Lys Lys Leu Val Phe Gly Tyr Arg Lys Pro
       210                 215                 220
   Leu Asp Asn Ile Lys Asp Asn Val Gly Lys Met Glu Asp Tyr Ile Lys
   225                 230                 235                 240
   Lys Asn Lys Thr Thr Ile Ala Asn Ile Asn Glu Leu Ile Glu Gly Ser
                   245                 250                 255
   Lys Lys Thr Ile Asp Gln Asn Lys Asn Ala Asp Asn Glu Glu Gly Lys
               260                 265                 270
   Lys Lys Leu Tyr Gln Ala Gln Tyr Asp Leu Ser Ile Tyr Asn Lys Gln
           275                 280                 285
   Leu Glu Glu Ala His Asn Leu Ile Ser Val Leu Glu Lys Arg Ile Asp
       290                 295                 300
   Thr Leu Lys Lys Asn Glu Asn Ile Lys Lys Leu Leu Asp Lys Ile Asn
   305                 310                 315                 320
   Glu Ile Lys Asn Pro Pro Pro Ala Asn Ser Gly Asn Thr Pro Asn Thr
                   325                 330                 335
   Leu Leu Asp Lys Asn Lys Lys Ile Glu Glu His Glu Glu Lys Ile Lys
               340                 345                 350
   Glu Ile Ala Lys Thr Ile Lys Phe Asn Ile Asp Ser Leu Phe Thr Asp
           355                 360                 365
   Pro Leu Glu Leu Glu Tyr Tyr Leu Arg Glu Lys Asn Lys Lys Val Asp
       370                 375                 380
   Val Thr Pro Lys Ser Gln Asp Pro Thr Lys Ser Val Gln Ile Pro Lys
   385                 390                 395                 400
   Val Pro Tyr Pro Asn Gly Ile Val Tyr Pro Leu Pro Leu Thr Asp Ile
                   405                 410                 415
   His Asn Ser Leu Ala Ala Asp Asn Asp Lys Asn Ser Tyr Gly Asp Leu
               420                 425                 430
   Met Asn Pro His Thr Lys Glu Lys Ile Asn Glu Lys Ile Ile Thr Asp
   435 440 445
   Asn Lys Glu Arg Lys Ile Phe Ile Asn Asn Ile Lys Lys Lys Ile Asp
   450 455 460
   Leu Glu Glu Lys Asn Ile Asn His Thr Lys Glu Gln Asn Lys Lys Leu
   465 470 475 480
   Leu Glu Asp Tyr Glu Lys Ser Lys Lys Asp Tyr Glu Glu Leu Leu Glu
   485 490 495
   Lys Phe Tyr Glu Met Lys Phe Asn Asn Asn Phe Asp Lys Asp Val Val
   500 505 510
   Asp Lys Ile Phe Ser Ala Arg Tyr Thr Tyr Asn Val Glu Lys Gln Arg
   515 520 525
   Tyr Asn Asn Lys Phe Ser Ser Ser Asn Asn Ser Val Tyr Asn Val Gln
   530 535 540
   Lys Leu Lys Lys Ala Leu Ser Tyr Leu Glu Asp Tyr Ser Leu Arg Lys
   545 550 555 560
   Gly Ile Ser Glu Lys Asp Phe Asn His Tyr Tyr Thr Leu Lys Thr Gly
   565 570 575
   Leu Glu Ala Asp Ile Lys Lys Leu Thr Glu Glu Ile Lys Ser Ser Glu
   580 585 590
   Asn Lys Ile Leu Glu Lys Asn Phe Lys Gly Leu Thr His Ser Ala Asn
   595 600 605
   Gly Ser Leu Glu Val Ser Asp Ile Val Lys Leu Gln Val Gln Lys Val
   610 615 620
   Leu Leu Ile Lys Lys Ile Glu Asp Leu Arg Lys Ile Glu Leu Phe Leu
   625 630 635 640
   Lys Asn Ala Gln Leu Lys Asp Ser Ile His Val Pro Asn Ile Tyr Lys
   645 650 655
   Pro Gln Asn Lys Pro Glu Pro Tyr Tyr Leu Ile Val Leu Lys Lys Glu
   660 665 670
   Val Asp Lys Leu Lys Glu Phe Ile Pro Lys Val Lys Asp Met Leu Lys
   675 680 685
   Lys Glu Gln Ala Val Leu Ser Ser Ile Thr Gln Pro Leu Val Ala Ala
   690 695 700
   Ser Glu Thr Thr Glu Asp Gly Gly His Ser Thr His Thr Leu Ser Gln
   705 710 715 720
   Ser Gly Glu Thr Glu Val Thr Glu Glu Thr Glu Glu Thr Glu Glu Thr
   725 730 735
   Val Gly His Thr Thr Thr Val Thr Ile Thr Leu Pro Pro Thr Gln Pro
   740 745 750
   Ser Pro Pro Lys Glu Val Lys Val Val Glu Asn Ser Ile Glu Gln Lys
   755 760 765
   Ser Asn Asp Asn Ser Gln Ala Leu Thr Lys Thr Val Tyr Leu Lys Lys
   770 775 780
   Leu Asp Glu Phe Leu Thr Lys Ser Tyr Ile Cys His Lys Tyr Ile Leu
   785 790 795 800
   Val Ser Asn Ser Ser Met Asp Gln Lys Leu Leu Glu Val Tyr Asn Leu
   805 810 815
   Thr Pro Glu Glu Glu Asn Glu Leu Lys Ser Cys Asp Pro Leu Asp Leu
   820 825 830
   Leu Phe Asn Ile Gln Asn Asn Ile Pro Ala Met Tyr Ser Leu Tyr Asp
   835 840 845
   Ser Met Asn Asn Asp Leu Gln His Leu Phe Phe Glu Leu Tyr Gln Lys
   850 855 860
   Glu Met Ile Tyr Tyr Leu His Lys Leu Lys Glu Glu Asn His Ile Lys
   865 870 875 880
   Lys Leu Leu Glu Glu Gln Lys Gln Ile Thr Gly Thr Ser
   885 890
<210> 2
   <211> 893
   <212> PRT
   <213> Artificial Sequence
<220>
   <223> p83/30 from P. falciparum strain 3D7 (amino acid mutations E612K and Q767H)
<400> 2
   Met Val Thr His Glu Ser Tyr Gln Glu Leu Val Lys Lys Leu Glu Ala
   1 5 10 15
   Leu Glu Asp Ala Val Leu Thr Gly Tyr Ser Leu Phe Gln Lys Glu Lys
   20 25 30
   Met Val Leu Asn Glu Glu Glu Ile Thr Thr Lys Gly Ala Ser Ala Gln
   35 40 45
   Ser Gly Ala Ser Ala Gln Ser Gly Ala Ser Ala Gln Ser Gly Ala Ser
   50 55 60
   Ala Gln Ser Gly Ala Ser Ala Gln Ser Gly Ala Ser Ala Gln Ser Gly
   65 70 75 80
   Thr Ser Gly Pro Ser Gly Pro Ser Gly Thr Ser Pro Ser Ser Arg Ser
   85 90 95
   Asn Thr Leu Pro Arg Ser Asn Thr Ser Ser Gly Ala Ser Pro Pro Ala
   100 105 110
   Asp Ala Ser Asp Ser Asp Ala Lys Ser Tyr Ala Asp Leu Lys His Arg
   115 120 125
   Val Arg Asn Tyr Leu Phe Thr Ile Lys Glu Leu Lys Tyr Pro Glu Leu
   130 135 140
   Phe Asp Leu Thr Asn His Met Leu Thr Leu Cys Asp Asn Ile His Gly
   145 150 155 160
   Phe Lys Tyr Leu Ile Asp Gly Tyr Glu Glu Ile Asn Glu Leu Leu Tyr
   165 170 175
   Lys Leu Asn Phe Tyr Phe Asp Leu Leu Arg Ala Lys Leu Asn Asp Val
   180 185 190
   Cys Ala Asn Asp Tyr Cys Gln Ile Pro Phe Asn Leu Lys Ile Arg Ala
   195 200 205
   Asn Glu Leu Asp Val Leu Lys Lys Leu Val Phe Gly Tyr Arg Lys Pro
   210 215 220
   Leu Asp Asn Ile Lys Asp Asn Val Gly Lys Met Glu Asp Tyr Ile Lys
   225 230 235 240
   Lys Asn Lys Thr Thr Ile Ala Asn Ile Asn Glu Leu Ile Glu Gly Ser
   245 250 255
   Lys Lys Thr Ile Asp Gln Asn Lys Asn Ala Asp Asn Glu Glu Gly Lys
   260 265 270
   Lys Lys Leu Tyr Gln Ala Gln Tyr Asp Leu Ser Ile Tyr Asn Lys Gln
   275 280 285
   Leu Glu Glu Ala His Asn Leu Ile Ser Val Leu Glu Lys Arg Ile Asp
   290 295 300
   Thr Leu Lys Lys Asn Glu Asn Ile Lys Lys Leu Leu Asp Lys Ile Asn
   305 310 315 320
   Glu Ile Lys Asn Pro Pro Pro Ala Asn Ser Gly Asn Thr Pro Asn Thr
   325 330 335
   Leu Leu Asp Lys Asn Lys Lys Ile Glu Glu His Glu Glu Lys Ile Lys
   340 345 350
   Glu Ile Ala Lys Thr Ile Lys Phe Asn Ile Asp Ser Leu Phe Thr Asp
   355 360 365
   Pro Leu Glu Leu Glu Tyr Tyr Leu Arg Glu Lys Asn Lys Lys Val Asp
   370 375 380
   Val Thr Pro Lys Ser Gln Asp Pro Thr Lys Ser Val Gln Ile Pro Lys
   385 390 395 400
   Val Pro Tyr Pro Asn Gly Ile Val Tyr Pro Leu Pro Leu Thr Asp Ile
   405 410 415
   His Asn Ser Leu Ala Ala Asp Asn Asp Lys Asn Ser Tyr Gly Asp Leu
   420 425 430
   Met Asn Pro His Thr Lys Glu Lys Ile Asn Glu Lys Ile Ile Thr Asp
   435 440 445
   Asn Lys Glu Arg Lys Ile Phe Ile Asn Asn Ile Lys Lys Lys Ile Asp
   450 455 460
   Leu Glu Glu Lys Asn Ile Asn His Thr Lys Glu Gln Asn Lys Lys Leu
   465 470 475 480
   Leu Glu Asp Tyr Glu Lys Ser Lys Lys Asp Tyr Glu Glu Leu Leu Glu
   485 490 495
   Lys Phe Tyr Glu Met Lys Phe Asn Asn Asn Phe Asp Lys Asp Val Val
   500 505 510
   Asp Lys Ile Phe Ser Ala Arg Tyr Thr Tyr Asn Val Glu Lys Gln Arg
   515 520 525
   Tyr Asn Asn Lys Phe Ser Ser Ser Asn Asn Ser Val Tyr Asn Val Gln
   530 535 540
   Lys Leu Lys Lys Ala Leu Ser Tyr Leu Glu Asp Tyr Ser Leu Arg Lys
   545 550 555 560
   Gly Ile Ser Glu Lys Asp Phe Asn His Tyr Tyr Thr Leu Lys Thr Gly
   565 570 575
   Leu Glu Ala Asp Ile Lys Lys Leu Thr Glu Glu Ile Lys Ser Ser Glu
   580 585 590
   Asn Lys Ile Leu Glu Lys Asn Phe Lys Gly Leu Thr His Ser Ala Asn
   595 600 605
   Gly Ser Leu Lys Val Ser Asp Ile Val Lys Leu Gln Val Gln Lys Val
   610 615 620
   Leu Leu Ile Lys Lys Ile Glu Asp Leu Arg Lys Ile Glu Leu Phe Leu
   625 630 635 640
   Lys Asn Ala Gln Leu Lys Asp Ser Ile His Val Pro Asn Ile Tyr Lys
   645 650 655
   Pro Gln Asn Lys Pro Glu Pro Tyr Tyr Leu Ile Val Leu Lys Lys Glu
   660 665 670
   Val Asp Lys Leu Lys Glu Phe Ile Pro Lys Val Lys Asp Met Leu Lys
   675 680 685
   Lys Glu Gln Ala Val Leu Ser Ser Ile Thr Gln Pro Leu Val Ala Ala
   690 695 700
   Ser Glu Thr Thr Glu Asp Gly Gly His Ser Thr His Thr Leu Ser Gln
   705 710 715 720
   Ser Gly Glu Thr Glu Val Thr Glu Glu Thr Glu Glu Thr Glu Glu Thr
   725 730 735
   Val Gly His Thr Thr Thr Val Thr Ile Thr Leu Pro Pro Thr Gln Pro
   740 745 750
   Ser Pro Pro Lys Glu Val Lys Val Val Glu Asn Ser Ile Glu His Lys
   755 760 765
   Ser Asn Asp Asn Ser Gln Ala Leu Thr Lys Thr Val Tyr Leu Lys Lys
   770 775 780
   Leu Asp Glu Phe Leu Thr Lys Ser Tyr Ile Cys His Lys Tyr Ile Leu
   785 790 795 800
   Val Ser Asn Ser Ser Met Asp Gln Lys Leu Leu Glu Val Tyr Asn Leu
   805 810 815
   Thr Pro Glu Glu Glu Asn Glu Leu Lys Ser Cys Asp Pro Leu Asp Leu
   820 825 830
   Leu Phe Asn Ile Gln Asn Asn Ile Pro Ala Met Tyr Ser Leu Tyr Asp
   835 840 845
   Ser Met Asn Asn Asp Leu Gln His Leu Phe Phe Glu Leu Tyr Gln Lys
   850 855 860
   Glu Met Ile Tyr Tyr Leu His Lys Leu Lys Glu Glu Asn His Ile Lys
   865 870 875 880
   Lys Leu Leu Glu Glu Gln Lys Gln Ile Thr Gly Thr Ser
   885 890
<210> 3
   <211> 891
   <212> PRT
   <213> Artificial Sequence
<220>
   <223> p83/30 from P. falciparum strain FCB-1
<400> 3
   Met Val Thr His Glu Ser Tyr Gln Glu Leu Val Lys Lys Leu Glu Ala
   1 5 10 15
   Leu Glu Asp Ala Val Leu Thr Gly Tyr Ser Leu Phe Gln Lys Glu Lys
   20 25 30
   Met Val Leu Asn Glu Gly Thr Ser Gly Thr Ala Val Thr Thr Ser Thr
   35 40 45
   Pro Gly Ser Lys Gly Ser Val Ala Ser Gly Gly Ser Gly Gly Ser Val
   50 55 60
   Ala Ser Gly Gly Ser Val Ala Ser Gly Gly Ser Val Ala Ser Gly Gly
   65 70 75 80
   Ser Val Ala Ser Gly Gly Ser Gly Asn Ser Arg Arg Thr Asn Pro Ser
   85 90 95
   Asp Asn Ser Ser Asp Ser Asp Ala Lys Ser Tyr Ala Asp Leu Lys His
   100 105 110
   Arg Val Arg Asn Tyr Leu Leu Thr Ile Lys Glu Leu Lys Tyr Pro Gln
   115 120 125
   Leu Phe Asp Leu Thr Asn His Met Leu Thr Leu Cys Asp Asn Ile His
   130 135 140
   Gly Phe Lys Tyr Leu Ile Asp Gly Tyr Glu Glu Ile Asn Glu Leu Leu
   145 150 155 160
   Tyr Lys Leu Asn Phe Tyr Phe Asp Leu Leu Arg Ala Lys Leu Asn Asp
   165 170 175
   Val Cys Ala Asn Asp Tyr Cys Gln Ile Pro Phe Asn Leu Lys Ile Arg
   180 185 190
   Ala Asn Glu Leu Asp Val Leu Lys Lys Leu Val Phe Gly Tyr Arg Lys
   195 200 205
   Pro Leu Asp Asn Ile Lys Asp Asn Val Gly Lys Met Glu Asp Tyr Ile
   210 215 220
   Lys Lys Asn Lys Lys Thr Ile Glu Asn Ile Asn Glu Leu Ile Glu Glu
   225 230 235 240
   Ser Lys Lys Thr Ile Asp Lys Asn Lys Asn Ala Thr Lys Glu Glu Glu
   245 250 255
   Lys Lys Lys Leu Tyr Gln Ala Gln Tyr Asp Leu Ser Ile Tyr Asn Lys
   260 265 270
   Gln Leu Glu Glu Ala His Asn Leu Ile Ser Val Leu Glu Lys Arg Ile
   275 280 285
   Asp Thr Leu Lys Lys Asn Glu Asn Ile Lys Glu Leu Leu Asp Lys Ile
   290 295 300
   Asn Glu Ile Lys Asn Pro Pro Pro Ala Asn Ser Gly Asn Thr Pro Asn
   305 310 315 320
   Thr Leu Leu Asp Lys Asn Lys Lys Ile Glu Glu His Glu Lys Glu Ile
   325 330 335
   Lys Glu Ile Ala Lys Thr Ile Lys Phe Asn Ile Asp Ser Leu Phe Thr
   340 345 350
   Asp Pro Leu Glu Leu Glu Tyr Tyr Leu Arg Glu Lys Asn Lys Asn Ile
   355 360 365
   Asp Ile Ser Ala Lys Val Glu Thr Lys Glu Ser Thr Glu Pro Asn Glu
   370 375 380
   Tyr Pro Asn Gly Val Thr Tyr Pro Leu Ser Tyr Asn Asp Ile Asn Asn
   385 390 395 400
   Ala Leu Asn Glu Leu Asn Ser Phe Gly Asp Leu Ile Asn Pro Phe Asp
   405 410 415
   Tyr Thr Lys Glu Pro Ser Lys Asn Ile Tyr Thr Asp Asn Glu Arg Lys
   420 425 430
   Lys Phe Ile Asn Glu Ile Lys Glu Lys Ile Lys Ile Glu Lys Lys Lys
   435 440 445
   Ile Glu Ser Asp Lys Lys Ser Tyr Glu Asp Arg Ser Lys Ser Leu Asn
   450 455 460
   Asp Ile Thr Lys Glu Tyr Glu Lys Leu Leu Asn Glu Ile Tyr Asp Ser
   465                 470                 475                 480
   Lys Phe Asn Asn Asn Ile Asp Leu Thr Asn Phe Glu Lys Met Met Gly
                   485                 490                 495
   Lys Arg Tyr Ser Tyr Lys Val Glu Lys Leu Thr His His Asn Thr Phe
               500                 505                 510
   Ala Ser Tyr Glu Asn Ser Lys His Asn Leu Glu Lys Leu Thr Lys Ala
           515                 520                 525
   Leu Lys Tyr Met Glu Asp Tyr Ser Leu Arg Asn Ile Val Val Glu Lys
       530                 535                 540
   Glu Leu Lys Tyr Tyr Lys Asn Leu Ile Ser Lys Ile Glu Asn Glu Ile
   545                 550                 555                 560
   Glu Thr Leu Val Glu Asn Ile Lys Lys Asp Glu Glu Gln Leu Phe Glu
                   565                 570                 575
   Lys Lys Ile Thr Lys Asp Glu Asn Lys Pro Asp Glu Lys Ile Leu Glu
               580                 585                 590
   Val Ser Asp Ile Val Lys Val Gln Val Gln Lys Val Leu Leu Met Asn
           595                 600                 605
   Lys Ile Asp Glu Leu Lys Lys Thr Gln Leu Ile Leu Lys Asn Val Glu
       610                 615                 620
   Leu Lys His Asn Ile His Val Pro Asn Ser Tyr Lys Gln Glu Asn Lys
   625                 630                 635                 640
   Gln Glu Pro Tyr Tyr Leu Ile Val Leu Lys Lys Glu Ile Asp Lys Leu
                   645                 650                 655
   Lys Val Phe Met Pro Lys Val Glu Ser Leu Ile Asn Glu Glu Lys Lys
               660                 665                 670
   Asn Ile Lys Thr Glu Gly Gln Ser Asp Asn Ser Glu Pro Ser Thr Glu
           675                 680                 685
   Gly Glu Ile Thr Gly Gln Ala Thr Thr Lys Pro Gly Gln Gln Ala Gly
       690                 695                 700
   Ser Ala Leu Glu Gly Asp Ser Val Gln Ala Gln Ala Gln Glu Gln Lys
   705                 710                 715                 720
   Gln Ala Gln Pro Pro Val Pro Val Pro Val Pro Glu Ala Lys Ala Gln
                   725                 730                 735
   Val Pro Thr Pro Pro Ala Pro Val Asn Asn Lys Thr Glu Asn Val Ser
   740 745 750
   Lys Leu Asp Tyr Leu Glu Lys Leu Tyr Glu Phe Leu Asn Thr Ser Tyr
   755 760 765
   Ile Cys His Lys Tyr Ile Leu Val Ser His Ser Thr Met Asn Glu Lys
   770 775 780
   Ile Leu Lys Gln Tyr Lys Ile Thr Lys Glu Glu Glu Ser Lys Leu Ser
   785 790 795 800
   Ser Cys Asp Pro Leu Asp Leu Leu Phe Asn Ile Gln Asn Asn Ile Pro
   805 810 815
   Val Met Tyr Ser Met Phe Asp Ser Leu Asn Asn Ser Leu Ser Gln Leu
   820 825 830
   Phe Met Glu Ile Tyr Glu Lys Glu Met Val Cys Asn Leu Tyr Lys Leu
   835 840 845
   Lys Asp Asn Asp Lys Ile Lys Asn Leu Leu Glu Glu Ala Lys Lys Val
   850 855 860
   Ser Thr Ser Val Lys Thr Leu Ser Ser Ser Ser Met Gln Pro Leu Ser
   865 870 875 880
   Leu Thr Pro Gln Asp Lys Pro Glu Val Ser Ala
   885 890
<210> 4
   <211> 794
   <212> PRT
   <213> Artificial Sequence
<220>
   <223> p38/42 from P. falciparum strain 3D7
<400> 4
   Met Ser Ser Thr Ser Ser Pro Gly Asn Thr Thr Val Asn Thr Ala Gln
   1 5 10 15
   Ser Ala Thr His Ser Asn Ser Gln Asn Gln Gln Ser Asn Ala Ser Ser
   20 25 30
   Thr Asn Thr Gln Asn Gly Val Ala Val Ser Ser Gly Pro Ala Val Val
   35 40 45
   Glu Glu Ser His Asp Pro Leu Thr Val Leu Ser Ile Ser Asn Asp Leu
   50 55 60
   Lys Gly Ile Val Ser Leu Leu Asn Leu Gly Asn Lys Thr Lys Val Pro
   65                  70                  75                  80
   Asn Pro Leu Thr Ile Ser Thr Thr Glu Met Glu Lys Phe Tyr Glu Asn
                   85                  90                  95
   Ile Leu Lys Asn Asn Asp Thr Tyr Phe Asn Asp Asp Ile Lys Gln Phe
               100                 105                 110
   Val Lys Ser Asn Ser Lys Val Ile Thr Gly Leu Thr Glu Thr Gln Lys
           115                 120                 125
   Asn Ala Leu Asn Asp Glu Ile Lys Lys Leu Lys Asp Thr Leu Gln Leu
       130                 135                 140
   Ser Phe Asp Leu Tyr Asn Lys Tyr Lys Leu Lys Leu Asp Arg Leu Phe
   145                 150                 155                 160
   Asn Lys Lys Lys Glu Leu Gly Gln Asp Lys Met Gln Ile Lys Lys Leu
                   165                 170                 175
   Thr Leu Leu Lys Glu Gln Leu Glu Ser Lys Leu Asn Ser Leu Asn Asn
               180                 185                 190
   Pro His Asn Val Leu Gln Asn Phe Ser Val Phe Phe Asn Lys Lys Lys
           195                 200                 205
   Glu Ala Glu Ile Ala Glu Thr Glu Asn Thr Leu Glu Asn Thr Lys Ile
       210                 215                 220
   Leu Leu Lys His Tyr Lys Gly Leu Val Lys Tyr Tyr Asn Gly Glu Ser
   225                 230                 235                 240
   Ser Pro Leu Lys Thr Leu Ser Glu Val Ser Ile Gln Thr Glu Asp Asn
                   245                 250                 255
   Tyr Ala Asn Leu Glu Lys Phe Arg Val Leu Ser Lys Ile Asp Gly Lys
               260                 265                 270
   Leu Asn Asp Asn Leu His Leu Gly Lys Lys Lys Leu Ser Phe Leu Ser
           275                 280                 285
   Ser Gly Leu His His Leu Ile Thr Glu Leu Lys Glu Val Ile Lys Asn
       290                 295                 300
   Lys Asn Tyr Thr Gly Asn Ser Pro Ser Glu Asn Asn Lys Lys Val Asn
   305                 310                 315                 320
   Glu Ala Leu Lys Ser Tyr Glu Asn Phe Leu Pro Glu Ala Lys Val Thr
                   325                 330                 335
   Thr Val Val Thr Pro Pro Gln Pro Asp Val Thr Pro Ser Pro Leu Ser
   340 345 350
   Val Arg Val Ser Gly Ser Ser Gly Ser Thr Lys Glu Glu Thr Gln Ile
   355 360 365
   Pro Thr Ser Gly Ser Leu Leu Thr Glu Leu Gln Gln Val Val Gln Leu
   370 375 380
   Gln Asn Tyr Asp Glu Glu Asp Asp Ser Leu Val Val Leu Pro Ile Phe
   385 390 395 400
   Gly Glu Ser Glu Asp Asn Asp Glu Tyr Leu Asp Gln Val Val Thr Gly
   405 410 415
   Glu Ala Ile Ser Val Thr Met Asp Asn Ile Leu Ser Gly Phe Glu Asn
   420 425 430
   Glu Tyr Asp Val Ile Tyr Leu Lys Pro Leu Ala Gly Val Tyr Arg Ser
   435 440 445
   Leu Lys Lys Gln Ile Glu Lys Asn Ile Phe Thr Phe Asn Leu Asn Leu
   450 455 460
   Asn Asp Ile Leu Asn Ser Arg Leu Lys Lys Arg Lys Tyr Phe Leu Asp
   465 470 475 480
   Val Leu Glu Ser Asp Leu Met Gln Phe Lys His Ile Ser Ser Asn Glu
   485 490 495
   Tyr Ile Ile Glu Asp Ser Phe Lys Leu Leu Asn Ser Glu Gln Lys Asn
   500 505 510
   Thr Leu Leu Lys Ser Tyr Lys Tyr Ile Lys Glu Ser Val Glu Asn Asp
   515 520 525
   Ile Lys Phe Ala Gln Glu Gly Ile Ser Tyr Tyr Glu Lys Val Leu Ala
   530 535 540
   Lys Tyr Lys Asp Asp Leu Glu Ser Ile Lys Lys Val Ile Lys Glu Glu
   545 550 555 560
   Lys Glu Lys Phe Pro Ser Ser Pro Pro Thr Thr Pro Pro Ser Pro Ala
   565 570 575
   Lys Thr Asp Glu Gln Lys Lys Glu Ser Lys Phe Leu Pro Phe Leu Thr
   580 585 590
   Asn Ile Glu Thr Leu Tyr Asn Asn Leu Val Asn Lys Ile Asp Asp Tyr
   595 600 605
   Leu Ile Asn Leu Lys Ala Lys Ile Asn Asp Cys Asn Val Glu Lys Asp
   610 615 620
   Glu Ala His Val Lys Ile Thr Lys Leu Ser Asp Leu Lys Ala Ile Asp
   625 630 635 640
   Asp Lys Ile Asp Leu Phe Lys Asn Pro Tyr Asp Phe Glu Ala Ile Lys
   645 650 655
   Lys Leu Ile Asn Asp Asp Thr Lys Lys Asp Met Leu Gly Lys Leu Leu
   660 665 670
   Ser Thr Gly Leu Val Gln Asn Phe Pro Asn Thr Ile Ile Ser Lys Leu
   675 680 685
   Ile Glu Gly Lys Phe Gln Asp Met Leu Asn Ile Ser Gln His Gln Cys
   690 695 700
   Val Lys Lys Gln Cys Pro Glu Asn Ser Gly Cys Phe Arg His Leu Asp
   705 710 715 720
   Glu Arg Glu Glu Cys Lys Cys Leu Leu Asn Tyr Lys Gln Glu Gly Asp
   725 730 735
   Lys Cys Val Glu Asn Pro Asn Pro Thr Cys Asn Glu Asn Asn Gly Gly
   740 745 750
   Cys Asp Ala Asp Ala Thr Cys Thr Glu Glu Asp Ser Gly Ser Ser Arg
   755 760 765
   Lys Lys Ile Thr Cys Glu Cys Thr Lys Pro Asp Ser Tyr Pro Leu Phe
   770 775 780
   Asp Gly Ile Phe Cys Ser Ser Ser Asn Val
   785 790
<210> 5
   <211> 713
   <212> PRT
   <213> Artificial Sequence
<220>
   <223> p38/42 from P. falciparum strain FCB-1
<400> 5
   Met Asn Asp Asp Thr Ser His Ser Thr Asn Leu Asn Asn Ser Leu Lys
   1 5 10 15
   Leu Phe Glu Asn Ile Leu Ser Leu Gly Lys Asn Lys Asn Ile Tyr Gln
   20 25 30
   Glu Leu Ile Gly Gln Lys Ser Ser Glu Asn Phe Tyr Glu Lys Ile Leu
   35 40 45
   Lys Asp Ser Asp Thr Phe Tyr Asn Glu Ser Phe Thr Asn Phe Val Lys
   50 55 60
   Ser Lys Ala Asp Asp Ile Asn Ser Leu Asn Asp Glu Ser Lys Arg Lys
   65 70 75 80
   Lys Leu Glu Glu Asp Ile Asn Lys Leu Lys Lys Thr Leu Gln Leu Ser
   85 90 95
   Phe Asp Leu Tyr Asn Lys Tyr Lys Leu Lys Leu Glu Arg Leu Phe Asp
   100 105 110
   Lys Lys Lys Thr Val Gly Lys Tyr Lys Met Gln Ile Lys Lys Leu Thr
   115 120 125
   Leu Leu Lys Glu Gln Leu Glu Ser Lys Leu Asn Ser Leu Asn Asn Pro
   130 135 140
   Lys His Val Leu Gln Asn Phe Ser Val Phe Phe Asn Lys Lys Lys Glu
   145 150 155 160
   Ala Glu Ile Ala Glu Thr Glu Asn Thr Leu Glu Asn Thr Lys Ile Leu
   165 170 175
   Leu Lys His Tyr Lys Gly Leu Val Lys Tyr Tyr Asn Gly Glu Ser Ser
   180 185 190
   Pro Leu Lys Thr Leu Ser Glu Glu Ser Ile Gln Thr Glu Asp Asn Tyr
   195 200 205
   Ala Ser Leu Glu Asn Phe Lys Val Leu Ser Lys Leu Glu Gly Lys Leu
   210 215 220
   Lys Asp Asn Leu Asn Leu Glu Lys Lys Lys Leu Ser Tyr Leu Ser Ser
   225 230 235 240
   Gly Leu His His Leu Ile Ala Glu Leu Lys Glu Val Ile Lys Asn Lys
   245 250 255
   Asn Tyr Thr Gly Asn Ser Pro Ser Glu Asn Asn Thr Asp Val Asn Asn
   260 265 270
   Ala Leu Glu Ser Tyr Lys Lys Phe Leu Pro Glu Gly Thr Asp Val Ala
   275 280 285
   Thr Val Val Ser Glu Ser Gly Ser Asp Thr Leu Glu Gln Ser Gln Pro
   290 295 300
   Lys Lys Pro Ala Ser Thr His Val Gly Ala Glu Ser Asn Thr Ile Thr
   305 310 315 320
   Thr Ser Gln Asn Val Asp Asp Glu Val Asp Asp Val Ile Ile Val Pro
   325 330 335
   Ile Phe Gly Glu Ser Glu Glu Asp Tyr Asp Asp Leu Gly Gln Val Val
   340 345 350
   Thr Gly Glu Ala Val Thr Pro Ser Val Ile Asp Asn Ile Leu Ser Lys
   355 360 365
   Ile Glu Asn Glu Tyr Glu Val Leu Tyr Leu Lys Pro Leu Ala Gly Val
   370 375 380
   Tyr Arg Ser Leu Lys Lys Gln Leu Glu Asn Asn Val Met Thr Phe Asn
   385 390 395 400
   Val Asn Val Lys Asp Ile Leu Asn Ser Arg Phe Asn Lys Arg Glu Asn
   405 410 415
   Phe Lys Asn Val Leu Glu Ser Asp Leu Ile Pro Tyr Lys Asp Leu Thr
   420 425 430
   Ser Ser Asn Tyr Val Val Lys Asp Pro Tyr Lys Phe Leu Asn Lys Glu
   435 440 445
   Lys Arg Asp Lys Phe Leu Ser Ser Tyr Asn Tyr Ile Lys Asp Ser Ile
   450 455 460
   Asp Thr Asp Ile Asn Phe Ala Asn Asp Val Leu Gly Tyr Tyr Lys Ile
   465 470 475 480
   Leu Ser Glu Lys Tyr Lys Ser Asp Leu Asp Ser Ile Lys Lys Tyr Ile
   485 490 495
   Asn Asp Lys Gln Gly Glu Asn Glu Lys Tyr Leu Pro Phe Leu Asn Asn
   500 505 510
   Ile Glu Thr Leu Tyr Lys Thr Val Asn Asp Lys Ile Asp Leu Phe Val
   515 520 525
   Ile His Leu Glu Ala Lys Val Leu Asn Tyr Thr Tyr Glu Lys Ser Asn
   530 535 540
   Val Glu Val Lys Ile Lys Glu Leu Asn Tyr Leu Lys Thr Ile Gln Asp
   545 550 555 560
   Lys Leu Ala Asp Phe Lys Lys Asn Asn Asn Phe Val Gly Ile Ala Asp
   565 570 575
   Leu Ser Thr Asp Tyr Asn His Asn Asn Leu Leu Thr Lys Phe Leu Ser
   580 585 590
   Thr Gly Met Val Phe Glu Asn Leu Ala Lys Thr Val Leu Ser Asn Leu
   595 600 605
   Leu Asp Gly Asn Leu Gln Gly Met Leu Asn Ile Ser Gln His Gln Cys
   610 615 620
   Val Lys Lys Gln Cys Pro Gln Asn Ser Gly Cys Phe Arg His Leu Asp
   625 630 635 640
   Glu Arg Glu Glu Cys Lys Cys Leu Leu Asn Tyr Lys Gln Glu Gly Asp
   645 650 655
   Lys Cys Val Glu Asn Pro Asn Pro Thr Cys Asn Glu Asn Asn Gly Gly
   660 665 670
   Cys Asp Ala Asp Ala Lys Cys Thr Glu Glu Asp Ser Gly Ser Asn Gly
   675 680 685
   Lys Lys Ile Thr Cys Glu Cys Thr Lys Pro Asp Ser Tyr Pro Leu Phe
   690 695 700
   Asp Gly Ile Phe Cys Ser Ser Ser Asn
   705 710
<210> 6
   <211> 4899
   <212> DNA
   <213> Artificial sequence
<220>
   <223> pZE23/d-83/30 (encoding SEQ ID 1; no silent mutations in RBS)
<400> 6
   ctcgagaaaa tttatcaaaa agagtgttga cttgtgagcg gataacaatg atacttagat 60
   tcaattgtga gcggataaca atttcacaca gaattcatta aagaggagaa aggatccatg 120
   gtgactcacg agagttatca ggaactcgtc aagaagctcg aagctttaga ggacgccgta 180
   ttgacaggtt actccctatt ccagaaagaa aagatggttt taaacgaaga agaaattacc 240
   acaaagggag catccgccca gtctggagca tctgctcaga gcggagcatc tgctcagagt 300
   ggagcaagcg cccaaagtgg agcgtctgcc cagtcaggcg cctcagctca atctggaacc 360
   tctgggccga gtggtcctag cggtacttct ccaagtagcc ggtctaatac actcccacgt 420
   tccaacacct ccagtggagc ctccccaccc gccgacgcat ccgactcaga cgctaagagt 480
   tatgcagacc tgaagcaccg cgtgaggaac taccttttca ctatcaaaga gttgaagtac 540
   cctgaattgt tcgatttgac caaccatatg ctgacactct gtgacaacat acatggtttc 600
   aagtatctga tagatgggta tgaagaaatt aacgagctgc tctataaact caacttttac 660
   ttcgacctgc tgcgtgccaa gctgaacgat gtctgtgcaa acgattactg ccagatccca 720
   ttcaacctaa agatacgtgc gaacgagctg gatgttctga agaaactcgt gttcgggtat 780
   cggaaaccct tggacaacat taaggacaat gtggggaaga tggaggatta cattaagaaa   840
   aataaaacaa caatcgctaa cataaatgag cttatcgagg ggagcaaaaa gaccatcgac   900
   cagaacaaga atgccgacaa tgaagaggga aaaaagaaac tataccaagc ccagtatgat   960
   ttgagcatct acaataagca actagaggaa gctcacaacc tcatcagcgt actggaaaag  1020
   agaattgaca ccctgaaaaa gaatgaaaac attaagaaac tcctggacaa gattaacgaa  1080
   attaaaaacc cacctccagc gaatagcgga aataccccga ataccctgct ggataagaac  1140
   aaaaagattg aagagcacga agagaaaatc aaggaaatcg ccaagactat taagttcaat  1200
   atagattctc tgttcacaga ccctctggag ctggaatact acctgcgcga gaagaataag  1260
   aaggtcgacg tgaccccaaa gagccaagac ccaacaaagt ccgtgcagat ccccaaagtg  1320
   ccctacccaa acggcatcgt gtatcccctg cctcttaccg acatccacaa ctctctggca  1380
   gccgataacg acaaaaacag ctatggagac ctgatgaacc cccacactaa ggaaaagata  1440
   aacgagaaga tcattaccga taataaggag cggaagattt ttatcaacaa catcaagaag  1500
   aaaatcgacc tggaagagaa aaatatcaat cacaccaaag agcaaaacaa gaaattactg  1560
   gaggactatg agaagagcaa aaaggattat gaggaactgt tagagaagtt ctatgaaatg  1620
   aaattcaaca acaatttcga taaggatgtg gtcgataaaa ttttcagcgc ccggtacacc  1680
   tacaacgtgg agaagcagcg gtacaacaat aagttcagca gctccaataa ctcggtctac  1740
   aatgtgcaga agctgaagaa agctctgagc tatctggaag actactcgct gaggaaaggg  1800
   atttctgaga aggatttcaa ccactactac accctcaaaa ccggcctgga agctgacatc  1860
   aagaaactca ctgaagagat caaaagttct gagaataaga tactggagaa gaacttcaag  1920
   ggactaacgc actctgcaaa cggctccctg gaagtctctg acatcgtgaa actgcaagtc  1980
   caaaaggtgc tgctcatcaa aaaaatcgag gatctgcgaa agatcgagct gtttcttaag  2040
   aacgcccaac tgaaagactc aatccacgtg cctaacattt acaaaccgca gaacaaacca  2100
   gaaccatact atctgatcgt gctgaagaag gaggtggata agctgaagga attcatccca  2160
   aaagtgaaag atatgttaaa gaaagagcaa gccgtgctga gcagcataac gcagcctctg  2220
   gtggccgcaa gcgagacaac cgaagatggc gggcacagca cccacaccct gtctcagtct  2280
   ggcgaaacag aggtgacaga agagacagaa gagaccgaag aaacagtggg gcacaccact  2340
   actgtgacca tcactttgcc ccctacgcag ccatctcccc caaaagaggt caaagtcgtg  2400
   gaaaactcca ttgaacagaa gtccaacgac aactcacagg ctctgacgaa gaccgtctat  2460
   ctgaagaaac tggacgagtt cctgaccaaa agctacatct gccataaata catcctcgtg  2520
   tctaacagca gcatggatca gaagctgttg gaggtgtaca acctaacgcc cgaagaagag  2580
   aacgagttaa aatcctgtga tcccttagac ctactgttta acattcagaa caacatcccc  2640
   gctatgtaca gcttatatga ttccatgaat aacgacctcc agcacctgtt cttcgagctg  2700
   taccagaaag agatgatcta ctatctgcat aagctgaaag aggagaatca catcaaaaag  2760
   ttgctggaag agcagaaaca gataactggg acgtcctaat agcgcatcga taagctagct  2820
   taattagctg atctagaggc atcaaataaa acgaaaggct cagtcgaaag actgggcctt 2880
   tcgttttatc tgttgtttgt cggtgaacgc tctcctgagt aggacaaatc cgccgcccta 2940
   gacctaggcg ttcggctgcg gcgagcggta tcagctcact caaaggcggt aatacggtta 3000
   tccacagaat caggggataa cgcaggaaag aacatgtgag caaaaggcca gcaaaaggcc 3060
   aggaaccgta aaaaggccgc gttgctggcg tttttccata ggctccgccc ccctgacgag 3120
   catcacaaaa atcgacgctc aagtcagagg tggcgaaacc cgacaggact ataaagatac 3180
   caggcgtttc cccctggaag ctccctcgtg cgctctcctg ttccgaccct gccgcttacc 3240
   ggatacctgt ccgcctttct cccttcggga agcgtggcgc tttctcaatg ctcacgctgt 3300
   aggtatctca gttcggtgta ggtcgttcgc tccaagctgg gctgtgtgca cgaacccccc 3360
   gttcagcccg accgctgcgc cttatccggt aactatcgtc ttgagtccaa cccggtaaga 3420
   cacgacttat cgccactggc agcagccact ggtaacagga ttagcagagc gaggtatgta 3480
   ggcggtgcta cagagttctt gaagtggtgg cctaactacg gctacactag aaggacagta 3540
   tttggtatct gcgctctgct gaagccagtt accttcggaa aaagagttgg tagctcttga 3600
   tccggcaaac aaaccaccgc tggtagcggt ggtttttttg tttgcaagca gcagattacg 3660
   cgcagaaaaa aaggatctca agaagatcct ttgatctttt ctacggggtc tgacgctcag 3720
   tggaacgaaa actcacgtta agggattttg gtcatgacta gtgcttggat tctcaccaat 3780
   aaaaaacgcc cggcggcaac cgagcgttct gaacaaatcc agatggagtt ctgaggtcat 3840
   tactggatct atcaacagga gtccaagcga gctctcgaac cccagagtcc cgctcagaag 3900
   aactcgtcaa gaaggcgata gaaggcgatg cgctgcgaat cgggagcggc gataccgtaa 3960
   agcacgagga agcggtcagc ccattcgccg ccaagctctt cagcaatatc acgggtagcc 4020
   aacgctatgt cctgatagcg gtccgccaca cccagccggc cacagtcgat gaatccagaa 4080
   aagcggccat tttccaccat gatattcggc aagcaggcat cgccatgggt cacgacgaga 4140
   tcctcgccgt cgggcatgcg cgccttgagc ctggcgaaca gttcggctgg cgcgagcccc 4200
   tgatgctctt cgtccagatc atcctgatcg acaagaccgg cttccatccg agtacgtgct 4260
   cgctcgatgc gatgtttcgc ttggtggtcg aatgggcagg tagccggatc aagcgtatgc 4320
   agccgccgca ttgcatcagc catgatggat actttctcgg caggagcaag gtgagatgac 4380
   aggagatcct gccccggcac ttcgcccaat agcagccagt cccttcccgc ttcagtgaca 4440
   acgtcgagca cagctgcgca aggaacgccc gtcgtggcca gccacgatag ccgcgctgcc 4500
   tcgtcctgca gttcattcag ggcaccggac aggtcggtct tgacaaaaag aaccgggcgc 4560
   ccctgcgctg acagccggaa cacggcggca tcagagcagc cgattgtctg ttgtgcccag 4620
   tcatagccga atagcctctc cacccaagcg gccggagaac ctgcgtgcaa tccatcttgt 4680
   tcaatcatgc gaaacgatcc tcatcctgtc tcttgatcag atcttgatcc cctgcgccat 4740
   cagatccttg gcggcaagaa agccatccag tttactttgc agggcttccc aaccttacca 4800
   gagggcgccc cagctggcaa ttccgacgtc taagaaacca ttattatcat gacattaacc 4860
   tataaaaata ggcgtatcac gaggcccttt cgtcttcac 4899
<210> 7
   <211> 4899
   <212> DNA
   <213> Artificial Sequence
<220>
   <223> pZE23/d-83/30 (encoding SEQ ID 2; no silent mutations in RBS)
<400> 7
   ctcgagaaaa tttatcaaaa agagtgttga cttgtgagcg gataacaatg atacttagat 60
   tcaattgtga gcggataaca atttcacaca gaattcatta aagaggagaa aggatccatg 120
   gtgactcacg agagttatca ggaactcgtc aagaagctcg aagctttaga ggacgccgta 180
   ttgacaggtt actccctatt ccagaaagaa aagatggttt taaacgaaga agaaattacc 240
   acaaagggag catccgccca gtctggagca tctgctcaga gcggagcatc tgctcagagt 300
   ggagcaagcg cccaaagtgg agcgtctgcc cagtcaggcg cctcagctca atctggaacc 360
   tctgggccga gtggtcctag cggtacttct ccaagtagcc ggtctaatac actcccacgt 420
   tccaacacct ccagtggagc ctccccaccc gccgacgcat ccgactcaga cgctaagagt 480
   tatgcagacc tgaagcaccg cgtgaggaac taccttttca ctatcaaaga gttgaagtac 540
   cctgaattgt tcgatttgac caaccatatg ctgacactct gtgacaacat acatggtttc 600
   aagtatctga tagatgggta tgaagaaatt aacgagctgc tctataaact caacttttac 660
   ttcgacctgc tgcgtgccaa gctgaacgat gtctgtgcaa acgattactg ccagatccca 720
   ttcaacctaa agatacgtgc gaacgagctg gatgttctga agaaactcgt gttcgggtat 780
   cggaaaccct tggacaacat taaggacaat gtggggaaga tggaggatta cattaagaaa 840
   aataaaacaa caatcgctaa cataaatgag cttatcgagg ggagcaaaaa gaccatcgac 900
   cagaacaaga atgccgacaa tgaagaggga aaaaagaaac tataccaagc ccagtatgat 960
   ttgagcatct acaataagca actagaggaa gctcacaacc tcatcagcgt actggaaaag 1020
   agaattgaca ccctgaaaaa gaatgaaaac attaagaaac tcctggacaa gattaacgaa 1080
   attaaaaacc cacctccagc gaatagcgga aataccccga ataccctgct ggataagaac 1140
   aaaaagattg aagagcacga agagaaaatc aaggaaatcg ccaagactat taagttcaat 1200
   atagattctc tgttcacaga ccctctggag ctggaatact acctgcgcga gaagaataag 1260
   aaggtcgacg tgaccccaaa gagccaagac ccaacaaagt ccgtgcagat ccccaaagtg 1320
   ccctacccaa acggcatcgt gtatcccctg cctcttaccg acatccacaa ctctctggca 1380
   gccgataacg acaaaaacag ctatggagac ctgatgaacc cccacactaa ggaaaagata 1440
   aacgagaaga tcattaccga taataaggag cggaagattt ttatcaacaa catcaagaag 1500
   aaaatcgacc tggaagagaa aaatatcaat cacaccaaag agcaaaacaa gaaattactg 1560
   gaggactatg agaagagcaa aaaggattat gaggaactgt tagagaagtt ctatgaaatg 1620
   aaattcaaca acaatttcga taaggatgtg gtcgataaaa ttttcagcgc ccggtacacc 1680
   tacaacgtgg agaagcagcg gtacaacaat aagttcagca gctccaataa ctcggtctac 1740
   aatgtgcaga agctgaagaa agctctgagc tatctggaag actactcgct gaggaaaggg 1800
   atttctgaga aggatttcaa ccactactac accctcaaaa ccggcctgga agctgacatc 1860
   aagaaactca ctgaagagat caaaagttct gagaataaga tactggagaa gaacttcaag 1920
   ggactaacgc actctgcaaa cggctccctg aaagtctctg acatcgtgaa actgcaagtc 1980
   caaaaggtgc tgctcatcaa aaaaatcgag gatctgcgaa agatcgagct gtttcttaag 2040
   aacgcccaac tgaaagactc aatccacgtg cctaacattt acaaaccgca gaacaaacca 2100
   gaaccatact atctgatcgt gctgaagaag gaggtggata agctgaagga attcatccca 2160
   aaagtgaaag atatgttaaa gaaagagcaa gccgtgctga gcagcataac gcagcctctg 2220
   gtggccgcaa gcgagacaac cgaagatggc gggcacagca cccacaccct gtctcagtct 2280
   ggcgaaacag aggtgacaga agagacagaa gagaccgaag aaacagtggg gcacaccact 2340
   actgtgacca tcactttgcc ccctacgcag ccatctcccc caaaagaggt caaagtcgtg 2400
   gaaaactcca ttgaacacaa gtccaacgac aactcacagg ctctgacgaa gaccgtctat 2460
   ctgaagaaac tggacgagtt cctgaccaaa agctacatct gccataaata catcctcgtg 2520
   tctaacagca gcatggatca gaagctgttg gaggtgtaca acctaacgcc cgaagaagag 2580
   aacgagttaa aatcctgtga tcccttagac ctactgttta acattcagaa caacatcccc 2640
   gctatgtaca gcttatatga ttccatgaat aacgacctcc agcacctgtt cttcgagctg 2700
   taccagaaag agatgatcta ctatctgcat aagctgaaag aggagaatca catcaaaaag 2760
   ttgctggaag agcagaaaca gataactggg acgtcctaat agcgcatcga taagctagct 2820
   taattagctg atctagaggc atcaaataaa acgaaaggct cagtcgaaag actgggcctt 2880
   tcgttttatc tgttgtttgt cggtgaacgc tctcctgagt aggacaaatc cgccgcccta 2940
   gacctaggcg ttcggctgcg gcgagcggta tcagctcact caaaggcggt aatacggtta 3000
   tccacagaat caggggataa cgcaggaaag aacatgtgag caaaaggcca gcaaaaggcc 3060
   aggaaccgta aaaaggccgc gttgctggcg tttttccata ggctccgccc ccctgacgag 3120
   catcacaaaa atcgacgctc aagtcagagg tggcgaaacc cgacaggact ataaagatac 3180
   caggcgtttc cccctggaag ctccctcgtg cgctctcctg ttccgaccct gccgcttacc 3240
   ggatacctgt ccgcctttct cccttcggga agcgtggcgc tttctcaatg ctcacgctgt 3300
   aggtatctca gttcggtgta ggtcgttcgc tccaagctgg gctgtgtgca cgaacccccc 3360
   gttcagcccg accgctgcgc cttatccggt aactatcgtc ttgagtccaa cccggtaaga 3420
   cacgacttat cgccactggc agcagccact ggtaacagga ttagcagagc gaggtatgta 3480
   ggcggtgcta cagagttctt gaagtggtgg cctaactacg gctacactag aaggacagta 3540
   tttggtatct gcgctctgct gaagccagtt accttcggaa aaagagttgg tagctcttga 3600
   tccggcaaac aaaccaccgc tggtagcggt ggtttttttg tttgcaagca gcagattacg 3660
   cgcagaaaaa aaggatctca agaagatcct ttgatctttt ctacggggtc tgacgctcag 3720
   tggaacgaaa actcacgtta agggattttg gtcatgacta gtgcttggat tctcaccaat 3780
   aaaaaacgcc cggcggcaac cgagcgttct gaacaaatcc agatggagtt ctgaggtcat 3840
   tactggatct atcaacagga gtccaagcga gctctcgaac cccagagtcc cgctcagaag 3900
   aactcgtcaa gaaggcgata gaaggcgatg cgctgcgaat cgggagcggc gataccgtaa 3960
   agcacgagga agcggtcagc ccattcgccg ccaagctctt cagcaatatc acgggtagcc 4020
   aacgctatgt cctgatagcg gtccgccaca cccagccggc cacagtcgat gaatccagaa 4080
   aagcggccat tttccaccat gatattcggc aagcaggcat cgccatgggt cacgacgaga 4140
   tcctcgccgt cgggcatgcg cgccttgagc ctggcgaaca gttcggctgg cgcgagcccc 4200
   tgatgctctt cgtccagatc atcctgatcg acaagaccgg cttccatccg agtacgtgct 4260
   cgctcgatgc gatgtttcgc ttggtggtcg aatgggcagg tagccggatc aagcgtatgc 4320
   agccgccgca ttgcatcagc catgatggat actttctcgg caggagcaag gtgagatgac 4380
   aggagatcct gccccggcac ttcgcccaat agcagccagt cccttcccgc ttcagtgaca 4440
   acgtcgagca cagctgcgca aggaacgccc gtcgtggcca gccacgatag ccgcgctgcc 4500
   tcgtcctgca gttcattcag ggcaccggac aggtcggtct tgacaaaaag aaccgggcgc 4560
   ccctgcgctg acagccggaa cacggcggca tcagagcagc cgattgtctg ttgtgcccag 4620
   tcatagccga atagcctctc cacccaagcg gccggagaac ctgcgtgcaa tccatcttgt 4680
   tcaatcatgc gaaacgatcc tcatcctgtc tcttgatcag atcttgatcc cctgcgccat 4740
   cagatccttg gcggcaagaa agccatccag tttactttgc agggcttccc aaccttacca 4800
   gagggcgccc cagctggcaa ttccgacgtc taagaaacca ttattatcat gacattaacc 4860
   tataaaaata ggcgtatcac gaggcccttt cgtcttcac 4899
<210> 8
   <211> 4899
   <212> DNA
   <213> Artificial sequence
<220>
   <223> pZE23/d-83/30 (encoding SEQ ID 1; with silent mutations in RBS)
<400> 8
   ctcgagaaaa tttatcaaaa agagtgttga cttgtgagcg gataacaatg atacttagat     60
   tcaattgtga gcggataaca atttcacaca gaattcatta aagaggagaa aggatccatg    120
   gtgactcacg aatcgtatca agaattggtg aagaagctcg aagctttaga ggacgccgta    180
   ttgacaggtt atagtttatt ccagaaagaa aagatggttt taaacgaaga agaaattacc    240
   acaaagggag catccgccca gtctggagca tctgctcaga gcggagcatc tgctcagagt    300
   ggagcaagcg cccaaagtgg agcgtctgcc cagtcaggcg cctcagctca atctggaacc    360
   tctgggccga gtggtcctag cggtacttct ccaagtagcc ggtctaatac actcccacgt    420
   tccaacacct ccagtggagc ctccccaccc gccgacgcat ccgactcaga cgctaagagt    480
   tatgcagacc tgaagcaccg cgtgaggaac taccttttca ctatcaaaga gttgaagtac    540
   cctgaattgt tcgatttgac caaccatatg ctgacactct gtgacaacat acatggtttc 600
   aagtatctga tagatgggta tgaagaaatt aacgagctgc tctataaact caacttttac 660
   ttcgacctgc tgcgtgccaa gctgaacgat gtctgtgcaa acgattactg ccagatccca 720
   ttcaacctaa agatacgtgc gaacgagctg gatgttctga agaaactcgt gttcgggtat 780
   cggaaaccct tggacaacat taaggacaat gtggggaaga tggaggatta cattaagaaa 840
   aataaaacaa caatcgctaa cataaatgag cttatcgagg ggagcaaaaa gaccatcgac 900
   cagaacaaga atgccgacaa tgaagaggga aaaaagaaac tataccaagc ccagtatgat 960
   ttgagcatct acaataagca actagaggaa gctcacaacc tcatcagcgt actggaaaag 1020
   agaattgaca ccctgaaaaa gaatgaaaac attaagaaac tcctggacaa gattaacgaa 1080
   attaaaaacc cacctccagc gaatagcgga aataccccga ataccctgct ggataagaac 1140
   aaaaagattg aagagcacga agagaaaatc aaggaaatcg ccaagactat taagttcaat 1200
   atagattctc tgttcacaga ccctctggag ctggaatact acctgcgcga gaagaataag 1260
   aaggtcgacg tgaccccaaa gagccaagac ccaacaaagt ccgtgcagat ccccaaagtg 1320
   ccctacccaa acggcatcgt gtatcccctg cctcttaccg acatccacaa ctctctggca 1380
   gccgataacg acaaaaacag ctatggagac ctgatgaacc cccacactaa ggaaaagata 1440
   aacgagaaga tcattaccga taataaggag cggaagattt ttatcaacaa catcaagaag 1500
   aaaatcgacc tggaagagaa aaatatcaat cacaccaaag agcaaaacaa gaaattactg 1560
   gaggactatg agaagagcaa aaaggattat gaggaactgt tagagaagtt ctatgaaatg 1620
   aaattcaaca acaatttcga taaggatgtg gtcgataaaa ttttcagcgc ccggtacacc 1680
   tacaacgtgg agaagcagcg gtacaacaat aagttcagca gctccaataa ctcggtctac 1740
   aatgtgcaga agctgaagaa agctctgagc tatctggaag actactcgct gaggaaaggg 1800
   atttctgaga aggatttcaa ccactactac accctcaaaa ccggcctgga agctgacatc 1860
   aagaaactca ctgaagagat caaaagttct gagaataaga tactggagaa gaacttcaag 1920
   ggactaacgc actctgcaaa cggctccctg gaagtctctg acatcgtgaa actgcaagtc 1980
   caaaaggtgc tgctcatcaa aaaaatcgag gatctgcgaa agatcgagct gtttcttaag 2040
   aacgcccaac tgaaagactc aatccacgtg cctaacattt acaaaccgca gaacaaacca 2100
   gaaccatact atctgatcgt gctgaagaag gaggtggata agctgaagga attcatccca 2160
   aaagtgaaag atatgttaaa gaaagagcaa gccgtgctga gcagcataac gcagcctctg 2220
   gtggccgcaa gcgagacaac cgaagatggc gggcacagca cccacaccct gtctcagtct 2280
   ggcgaaacag aggtgacaga agagacagaa gagaccgaag aaacagtggg gcacaccact 2340
   actgtgacca tcactttgcc ccctacgcag ccatctcccc caaaagaggt caaagtcgtg 2400
   gaaaactcca ttgaacagaa gtccaacgac aactcacagg ctctgacgaa gaccgtctat 2460
   ctgaagaaac tggacgagtt cctgaccaaa agctacatct gccataaata catcctcgtg 2520
   tctaacagca gcatggatca gaagctgttg gaggtgtaca acctaacgcc cgaagaagag 2580
   aacgagttaa aatcctgtga tcccttagac ctactgttta acattcagaa caacatcccc 2640
   gctatgtaca gcttatatga ttccatgaat aacgacctcc agcacctgtt cttcgagctg 2700
   taccagaaag agatgatcta ctatctgcat aagctgaaag aggagaatca catcaaaaag 2760
   ttgctggaag agcagaaaca gataactggg acgtcctaat agcgcatcga taagctagct 2820
   taattagctg atctagaggc atcaaataaa acgaaaggct cagtcgaaag actgggcctt 2880
   tcgttttatc tgttgtttgt cggtgaacgc tctcctgagt aggacaaatc cgccgcccta 2940
   gacctaggcg ttcggctgcg gcgagcggta tcagctcact caaaggcggt aatacggtta 3000
   tccacagaat caggggataa cgcaggaaag aacatgtgag caaaaggcca gcaaaaggcc 3060
   aggaaccgta aaaaggccgc gttgctggcg tttttccata ggctccgccc ccctgacgag 3120
   catcacaaaa atcgacgctc aagtcagagg tggcgaaacc cgacaggact ataaagatac 3180
   caggcgtttc cccctggaag ctccctcgtg cgctctcctg ttccgaccct gccgcttacc 3240
   ggatacctgt ccgcctttct cccttcggga agcgtggcgc tttctcaatg ctcacgctgt 3300
   aggtatctca gttcggtgta ggtcgttcgc tccaagctgg gctgtgtgca cgaacccccc 3360
   gttcagcccg accgctgcgc cttatccggt aactatcgtc ttgagtccaa cccggtaaga 3420
   cacgacttat cgccactggc agcagccact ggtaacagga ttagcagagc gaggtatgta 3480
   ggcggtgcta cagagttctt gaagtggtgg cctaactacg gctacactag aaggacagta 3540
   tttggtatct gcgctctgct gaagccagtt accttcggaa aaagagttgg tagctcttga 3600
   tccggcaaac aaaccaccgc tggtagcggt ggtttttttg tttgcaagca gcagattacg 3660
   cgcagaaaaa aaggatctca agaagatcct ttgatctttt ctacggggtc tgacgctcag 3720
   tggaacgaaa actcacgtta agggattttg gtcatgacta gtgcttggat tctcaccaat 3780
   aaaaaacgcc cggcggcaac cgagcgttct gaacaaatcc agatggagtt ctgaggtcat 3840
   tactggatct atcaacagga gtccaagcga gctctcgaac cccagagtcc cgctcagaag 3900
   aactcgtcaa gaaggcgata gaaggcgatg cgctgcgaat cgggagcggc gataccgtaa 3960
   agcacgagga agcggtcagc ccattcgccg ccaagctctt cagcaatatc acgggtagcc 4020
   aacgctatgt cctgatagcg gtccgccaca cccagccggc cacagtcgat gaatccagaa 4080
   aagcggccat tttccaccat gatattcggc aagcaggcat cgccatgggt cacgacgaga 4140
   tcctcgccgt cgggcatgcg cgccttgagc ctggcgaaca gttcggctgg cgcgagcccc 4200
   tgatgctctt cgtccagatc atcctgatcg acaagaccgg cttccatccg agtacgtgct 4260
   cgctcgatgc gatgtttcgc ttggtggtcg aatgggcagg tagccggatc aagcgtatgc 4320
   agccgccgca ttgcatcagc catgatggat actttctcgg caggagcaag gtgagatgac 4380
   aggagatcct gccccggcac ttcgcccaat agcagccagt cccttcccgc ttcagtgaca 4440
   acgtcgagca cagctgcgca aggaacgccc gtcgtggcca gccacgatag ccgcgctgcc 4500
   tcgtcctgca gttcattcag ggcaccggac aggtcggtct tgacaaaaag aaccgggcgc 4560
   ccctgcgctg acagccggaa cacggcggca tcagagcagc cgattgtctg ttgtgcccag 4620
   tcatagccga atagcctctc cacccaagcg gccggagaac ctgcgtgcaa tccatcttgt 4680
   tcaatcatgc gaaacgatcc tcatcctgtc tcttgatcag atcttgatcc cctgcgccat 4740
   cagatccttg gcggcaagaa agccatccag tttactttgc agggcttccc aaccttacca 4800
   gagggcgccc cagctggcaa ttccgacgtc taagaaacca ttattatcat gacattaacc 4860
   tataaaaata ggcgtatcac gaggcccttt cgtcttcac 4899
<210> 9
   <211> 4899
   <212> DNA
   <213> Artificial Sequence
<220>
   <223> pZE23/d-83/30 (encoding SEQ ID 2; with silent mutations in RBS)
<400> 9
   ctcgagaaaa tttatcaaaa agagtgttga cttgtgagcg gataacaatg atacttagat    60
   tcaattgtga gcggataaca atttcacaca gaattcatta aagaggagaa aggatccatg   120
   gtgactcacg aatcgtatca agaattggtg aagaagctcg aagctttaga ggacgccgta   180
   ttgacaggtt atagtttatt ccagaaagaa aagatggttt taaacgaaga agaaattacc   240
   acaaagggag catccgccca gtctggagca tctgctcaga gcggagcatc tgctcagagt   300
   ggagcaagcg cccaaagtgg agcgtctgcc cagtcaggcg cctcagctca atctggaacc   360
   tctgggccga gtggtcctag cggtacttct ccaagtagcc ggtctaatac actcccacgt   420
   tccaacacct ccagtggagc ctccccaccc gccgacgcat ccgactcaga cgctaagagt   480
   tatgcagacc tgaagcaccg cgtgaggaac taccttttca ctatcaaaga gttgaagtac   540
   cctgaattgt tcgatttgac caaccatatg ctgacactct gtgacaacat acatggtttc   600
   aagtatctga tagatgggta tgaagaaatt aacgagctgc tctataaact caacttttac   660
   ttcgacctgc tgcgtgccaa gctgaacgat gtctgtgcaa acgattactg ccagatccca   720
   ttcaacctaa agatacgtgc gaacgagctg gatgttctga agaaactcgt gttcgggtat   780
   cggaaaccct tggacaacat taaggacaat gtggggaaga tggaggatta cattaagaaa   840
   aataaaacaa caatcgctaa cataaatgag cttatcgagg ggagcaaaaa gaccatcgac   900
   cagaacaaga atgccgacaa tgaagaggga aaaaagaaac tataccaagc ccagtatgat   960
   ttgagcatct acaataagca actagaggaa gctcacaacc tcatcagcgt actggaaaag  1020
   agaattgaca ccctgaaaaa gaatgaaaac attaagaaac tcctggacaa gattaacgaa  1080
   attaaaaacc cacctccagc gaatagcgga aataccccga ataccctgct ggataagaac  1140
   aaaaagattg aagagcacga agagaaaatc aaggaaatcg ccaagactat taagttcaat  1200
   atagattctc tgttcacaga ccctctggag ctggaatact acctgcgcga gaagaataag  1260
   aaggtcgacg tgaccccaaa gagccaagac ccaacaaagt ccgtgcagat ccccaaagtg  1320
   ccctacccaa acggcatcgt gtatcccctg cctcttaccg acatccacaa ctctctggca  1380
   gccgataacg acaaaaacag ctatggagac ctgatgaacc cccacactaa ggaaaagata  1440
   aacgagaaga tcattaccga taataaggag cggaagattt ttatcaacaa catcaagaag 1500
   aaaatcgacc tggaagagaa aaatatcaat cacaccaaag agcaaaacaa gaaattactg 1560
   gaggactatg agaagagcaa aaaggattat gaggaactgt tagagaagtt ctatgaaatg 1620
   aaattcaaca acaatttcga taaggatgtg gtcgataaaa ttttcagcgc ccggtacacc 1680
   tacaacgtgg agaagcagcg gtacaacaat aagttcagca gctccaataa ctcggtctac 1740
   aatgtgcaga agctgaagaa agctctgagc tatctggaag actactcgct gaggaaaggg 1800
   atttctgaga aggatttcaa ccactactac accctcaaaa ccggcctgga agctgacatc 1860
   aagaaactca ctgaagagat caaaagttct gagaataaga tactggagaa gaacttcaag 1920
   ggactaacgc actctgcaaa cggctccctg aaagtctctg acatcgtgaa actgcaagtc 1980
   caaaaggtgc tgctcatcaa aaaaatcgag gatctgcgaa agatcgagct gtttcttaag 2040
   aacgcccaac tgaaagactc aatccacgtg cctaacattt acaaaccgca gaacaaacca 2100
   gaaccatact atctgatcgt gctgaagaag gaggtggata agctgaagga attcatccca 2160
   aaagtgaaag atatgttaaa gaaagagcaa gccgtgctga gcagcataac gcagcctctg 2220
   gtggccgcaa gcgagacaac cgaagatggc gggcacagca cccacaccct gtctcagtct 2280
   ggcgaaacag aggtgacaga agagacagaa gagaccgaag aaacagtggg gcacaccact 2340
   actgtgacca tcactttgcc ccctacgcag ccatctcccc caaaagaggt caaagtcgtg 2400
   gaaaactcca ttgaacacaa gtccaacgac aactcacagg ctctgacgaa gaccgtctat 2460
   ctgaagaaac tggacgagtt cctgaccaaa agctacatct gccataaata catcctcgtg 2520
   tctaacagca gcatggatca gaagctgttg gaggtgtaca acctaacgcc cgaagaagag 2580
   aacgagttaa aatcctgtga tcccttagac ctactgttta acattcagaa caacatcccc 2640
   gctatgtaca gcttatatga ttccatgaat aacgacctcc agcacctgtt cttcgagctg 2700
   taccagaaag agatgatcta ctatctgcat aagctgaaag aggagaatca catcaaaaag 2760
   ttgctggaag agcagaaaca gataactggg acgtcctaat agcgcatcga taagctagct 2820
   taattagctg atctagaggc atcaaataaa acgaaaggct cagtcgaaag actgggcctt 2880
   tcgttttatc tgttgtttgt cggtgaacgc tctcctgagt aggacaaatc cgccgcccta 2940
   gacctaggcg ttcggctgcg gcgagcggta tcagctcact caaaggcggt aatacggtta 3000
   tccacagaat caggggataa cgcaggaaag aacatgtgag caaaaggcca gcaaaaggcc 3060
   aggaaccgta aaaaggccgc gttgctggcg tttttccata ggctccgccc ccctgacgag 3120
   catcacaaaa atcgacgctc aagtcagagg tggcgaaacc cgacaggact ataaagatac 3180
   caggcgtttc cccctggaag ctccctcgtg cgctctcctg ttccgaccct gccgcttacc 3240
   ggatacctgt ccgcctttct cccttcggga agcgtggcgc tttctcaatg ctcacgctgt 3300
   aggtatctca gttcggtgta ggtcgttcgc tccaagctgg gctgtgtgca cgaacccccc 3360
   gttcagcccg accgctgcgc cttatccggt aactatcgtc ttgagtccaa cccggtaaga 3420
   cacgacttat cgccactggc agcagccact ggtaacagga ttagcagagc gaggtatgta 3480
   ggcggtgcta cagagttctt gaagtggtgg cctaactacg gctacactag aaggacagta 3540
   tttggtatct gcgctctgct gaagccagtt accttcggaa aaagagttgg tagctcttga 3600
   tccggcaaac aaaccaccgc tggtagcggt ggtttttttg tttgcaagca gcagattacg 3660
   cgcagaaaaa aaggatctca agaagatcct ttgatctttt ctacggggtc tgacgctcag 3720
   tggaacgaaa actcacgtta agggattttg gtcatgacta gtgcttggat tctcaccaat 3780
   aaaaaacgcc cggcggcaac cgagcgttct gaacaaatcc agatggagtt ctgaggtcat 3840
   tactggatct atcaacagga gtccaagcga gctctcgaac cccagagtcc cgctcagaag 3900
   aactcgtcaa gaaggcgata gaaggcgatg cgctgcgaat cgggagcggc gataccgtaa 3960
   agcacgagga agcggtcagc ccattcgccg ccaagctctt cagcaatatc acgggtagcc 4020
   aacgctatgt cctgatagcg gtccgccaca cccagccggc cacagtcgat gaatccagaa 4080
   aagcggccat tttccaccat gatattcggc aagcaggcat cgccatgggt cacgacgaga 4140
   tcctcgccgt cgggcatgcg cgccttgagc ctggcgaaca gttcggctgg cgcgagcccc 4200
   tgatgctctt cgtccagatc atcctgatcg acaagaccgg cttccatccg agtacgtgct 4260
   cgctcgatgc gatgtttcgc ttggtggtcg aatgggcagg tagccggatc aagcgtatgc 4320
   agccgccgca ttgcatcagc catgatggat actttctcgg caggagcaag gtgagatgac 4380
   aggagatcct gccccggcac ttcgcccaat agcagccagt cccttcccgc ttcagtgaca 4440
   acgtcgagca cagctgcgca aggaacgccc gtcgtggcca gccacgatag ccgcgctgcc 4500
   tcgtcctgca gttcattcag ggcaccggac aggtcggtct tgacaaaaag aaccgggcgc 4560
   ccctgcgctg acagccggaa cacggcggca tcagagcagc cgattgtctg ttgtgcccag 4620
   tcatagccga atagcctctc cacccaagcg gccggagaac ctgcgtgcaa tccatcttgt 4680
   tcaatcatgc gaaacgatcc tcatcctgtc tcttgatcag atcttgatcc cctgcgccat 4740
   cagatccttg gcggcaagaa agccatccag tttactttgc agggcttccc aaccttacca 4800
   gagggcgccc cagctggcaa ttccgacgtc taagaaacca ttattatcat gacattaacc 4860
   tataaaaata ggcgtatcac gaggcccttt cgtcttcac 4899
<210> 10
   <217> 4599
   <212> DNA
   <213> Artificial Sequence
<220>
   <223> pZE23/d-38/42 (encoding SEQ ID 4)
<400> 10

## Description

The invention relates to a method of preparing a modified nucleic acid encoding the p83/30 protein subunit of the merozoite surface protein 1 (MSP-1) from Plasmodium, corresponding modified nucleic acids, vectors comprising the same, a method of preparing the p83/30 fragment of MSP-1, uses of the above nucleic acid for making medicaments for preventing and/or treating malaria and a vaccine comprising any of the above nucleic acid or vector

Malaria is one of the most devastating infectious diseases in the world. According to estimates from the World Health Organisation (WHO) 400 to 900 million incidences of the disease are registered annually. According to information from the Multilateral Initiative against Malaria (MIM) between 700,000 and 2.7 million people die each year from the infection (MIM, 2001). In this respect 40% of the world's population in 99 countries are put at risk from malaria. The disease is caused by single-cell protozoa of the genus *Plasmodium* from the phylum *Apicomplexa.* There are four species which infect humans: *Plasmodium malariae,* responsible for Malaria quartana, *Plasmodium vivax* and *Plasmodium ovale,* both of which cause Malaria tertiana, and finally *Plasmodium falciparum,* the pathogen of Malaria tropica and responsible for almost all fatal infections.

Malaria is again currently spreading to an increasing extent. This is primarily attributable to intensive resistance formation of the malaria pathogen which is promoted in that the medicaments used for the therapy are also recommended and used for prophylaxis. Apart from the search for new chemotherapeutics, research is concentrating on the development of vaccines, because in the course of malaria infections in humans, immunity mechanisms are applied, a fact which is expressed in an increased resistance to the plasmodia, as demonstrated in the development of various types of immunity in humans in regions where malaria epidemics prevail.

In this context, WO 2004/038024 A describes MVA-based recombinant viruses comprising at least one nucleic acid coding for the *Plasmodium falciparum* MSP-1 protein, a fragment or a mutein thereof. Further, EP 1 637 602 describes a method for the recombinant production of a complete malaria antigen gp190/MSP-1 as well as parts thereof by expressing a synthetic DNA sequence. Finally, Kauth *et al.* (Kauth, Ch. W. et al., The Merozoite Surface Protein 1 Complex of Human Malaria Parasite Plasodium Falciparum, The Journal of Biological Chemistry, 278(25), 2003, pp. 22257-22264) describes the *in vitro* reconstitution of the MSP-1 complex of *Plasmodium falciparum* strain 3D7 from its heterologously produced subunits.

MSP-1 is the main surface protein of merozoites, the invasive form of the blood phases of the malaria pathogen. It is synthesized as a 190-220 kDa precursor which is fixed at the parasite surface via a glycosylphosphatidyl anchor. This protein is processed later during the development of the merozoites into smaller protein fragments which, however, remain non-covalently associated at the merozoite surface up to the invasion of erythrocytes by the parasites.

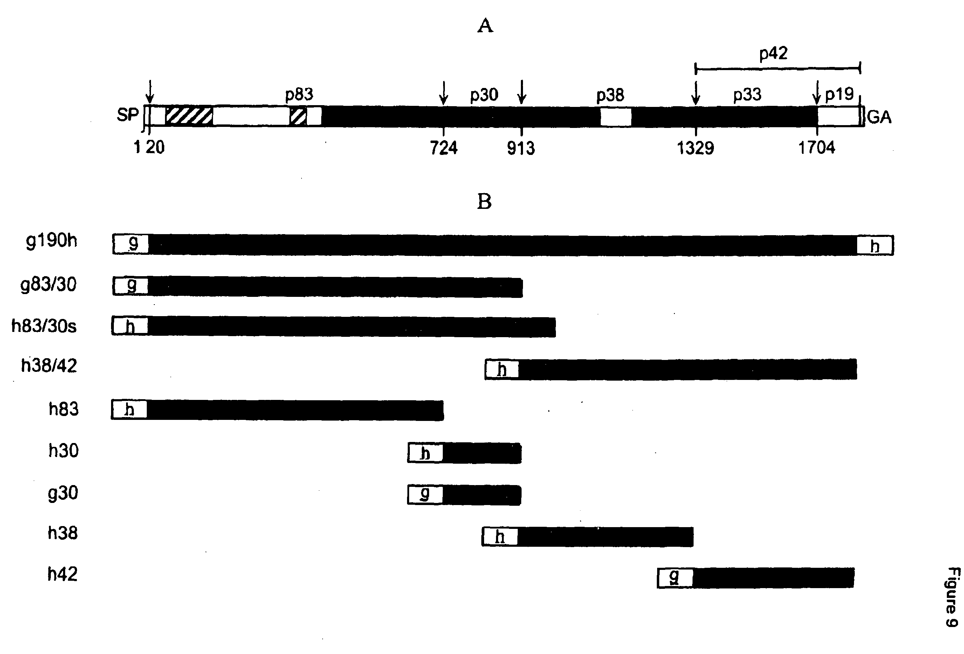

The sequences of the MSP-1 proteins of various *P*. *falciparum* strains fall into two groups, which have been named after two representative (prototypic) isolates Kᵢ and MAD20. Overall the protein consists of a number of highly preserved regions, dimorphic regions which allow the assignment of a given version of MSP-1 to one of the representative prototypes, and two relatively small oligomorphic blocks in the N-terminal part of the protein (Fig. 9 herein; Tanabe et al. (1987) J. Mol. Biol. 195, 273-87).

The immunization of mice with the protein purified from *P*. *yoelii* parasites protected the animals from the otherwise fatal infection. Also, the transfer of monoclonal antibodies against MSP-1 from *P. yoelii* provided protection in the mouse model.

Apart from studies on mice, Saimiri and Aotus monkeys have also been immunised with native, immune-affinity purified MSP-1. In these tests the protein obtained from *P. falciparum* partially provided either partial or complete protection against the ensuing infection by the parasite.

Purification of native material from Plasmodia is however expensive and this material cannot be used for production on a large scale. Therefore research into vaccines is concentrated on the development of recombinant vaccines.

For example, mice have been successfully immunized with MSP-1-19 purified from *E*. *coli* or *Saccharomyces cerevisiae* (Daly and Long (1993) Infect. Immun. 61, 2462-7; Ling et al. (1994) Parasite Immun. 16, 63-7; Tian et al. (1996) J. Immunol. 157, 1176-83; Hirunpetcharat et al (1997) J. Immunol. 15, 3400-11), as well as *Mycobacterium bovis* carrying MSP-1-19 (Matsumoto et al. (1999) Vaccine 18, 832-4). Alternatively to immunization with native or recombinant proteins, DNA coding for MSP-1-19 has also been used as a vaccine and protected immunized mice against infection with *P. chabaudi* (Wunderlich et al. (2000) Infect. Immun. 68, 5839-45).

In Phase I and II studies with MSP-1 fragments as vaccines, their immunogeneity was also shown in humans. In this respect p19 from *P. falciparum* fused onto a T-helper cell epitope of tetanus toxin (Keitel et al. (1999) Vaccine 18, 531-9) and the MSP-1 blocks 3 and 4 (Saul et al. (1999) Vaccine 17, 3145-59; Genton et al. (2000) Vaccine 18, 2504-11) are involved.

Some epidemiological studies in endemic regions show with adults a correlation between antibody titers against MSP-1 and the immunity against malaria (Tolle et al. (1993) Infect. Immun. 61, 40-7; Riley et al. (1992) Parasite Immunol. 14, 321-37; Riley et al. (1993) Parasite Immunol. 15, 513-24). These investigations together with the immunization studies on animals prove that MSP-1 is a promising candidate for the development of a malaria vaccine.

Generally, these studies can be differentiated into two approaches. Either purified material from parasites or material obtained in heterologous systems may be used.

Both for functional investigations and also for use as a vaccine, proteins must be produced reproducibly in a good yield and high quality. MSP-1 can be purified from parasites, but this is only possible on a small scale and with great expense and therefore cannot be carried out for obtaining MSP-1 according to the stated criteria in this way.

Heterologous production of full-length (190 kD) MSP-1 protein is extremely inefficient. Although this full-length protein contains the four major processing fragments p83, p30, p38 and p42 which, after proteolytic processing, form the non-covalently associated hotarotetramer, the extremely large size makes efficient heterologous production difficult, severely limiting obtainable yield. Specifically, the high AT-content of 76-78% of the respective parasite genes prevents stable cloning and heterologous expression in good yield. This problem has been solved by synthetic genes encoding the proteins in different codon compositions. Further, recovering a 190 kD protein containing numerous disulfide bonds in its proper conformation, for example from *E. coli,* is difficult experimentally, and wholly unsuited to technical scale preparation required for use of this protein as a vaccine.

Since MSP-1 is not glycosolated in the parasite despite numerous potential glycosolation sites, a prokaryotic expression system would be of advantage for technical scale preparation. However, the folding problems due to multiple disulfide bonds mentioned above persist. Kauth et al. (2003) (Journal of Biological Chemistry 278, 2257-2264) describes the construction of a heteromultimeric MSP-1 complex composed of recombinantly produced MSP-1 protein subunits. Here, synthetic polynucleotides encoding subunits and processing fragments of the MSP-1 complex of *P. falciparum* from strain 3D7 are used to generate each of the four individual MSP-1 subunits individually in highly purified and soluble form from *E. coli.* These subunits are then reassembled to form the MSP-1 heterotetramer. Kauth et al. (2003) describe production of the MSP-1 protein subunits p83, p30, p38 and p42 as well as two synthetic subunit "halves" p83/30 and p38/42. The subunit half p83/30 contains the amino acid sequences of the p83 and p30 subunits, linked into a single contiguous polypeptide. Likewise, the subunit half p38/42 contains the amino acid sequences of the p38 and p42 subunits, linked into a single contiguous polypeptide. While Kauth et al. indicates that stable production of MSP-1 protein for the purposes described above may best proceed via production of a heteromultimer, especially a heterodimer of the p83/30 and p38/42 subunits, the heteromultimeric proteins obtained in Kauth et al. (2003) remain ill-suited for the intended application as a vaccine.

First, the MSP-1 subunits of Kauth et al. were labeled with either His₆- or GST-tags so that they could be efficiently purified. These tags were required to isolate a sufficient amount of the subunit in high purity. Such tags are problematic from a regulatory standpoint, and post-expression proteolytic removal of the tags causes great loss in product. Thus, omitting protein tag would comply with regulatory requirements, but would result in a protein which is difficult if not impossible to purify. On the other hand, retaining the protein tag allows purification but precludes any use of the resulting protein as an approved therapeutic/vaccine.

A further problem encountered when constructing a heterodimer of p83/30 and p38/42 in a manner strictly analogous to Kauth et al. is that production of the p83/30 subunit in *E. coli* is very inefficient. Even using an isolation tag, only very low levels of p83/30 are recovered. Moreover, the small amount of the p83/30 fragment produced to begin with is attenuated further still by potent degradation *in vivo.*

There is thus a need for MSP-1 which is efficiently producible, complies with regulatory requirements set for therapeutic proteins, remains isolable in the high yield required for technical preparation and allows the generation of a strong immune response against *P*. *falciparum.* This need is met by the present invention.

One aspect of the invention provides a method for preparing a modified nucleic acid encoding the p83/30 fragment of MSP-1 of *Plasmodium* having an amino acid sequence as set out in SEQ ID NO: 1, 2 or 3, said method comprising:
a) Providing an mRNA sequence encoding the p83/30 fragment of MSP-1 from *Plasmodium* having an amino acid sequence as set out in SEQ ID NO: 1, 2 or 3;
b) Subjecting the region surrounding the start codon of the mRNA sequence of step (a) to analysis *in silico* to determine the predicted secondary structure of said mRNA sequence;
c) Identifying at least one stable, hairpin-like structure in said region surrounding the start codon of step (b);
d) Modifying the sequence of the mRNA in said region surrounding the start codon of step (b), wherein at least one nucleotide substitution, insertion, deletion and/or inversion destabilizes said hairpin-like region, and wherein the at least one nucleotide substitution, insertion, deletion and/or inversion is effected *in silico* so as to encode the same amino acid sequence as encoded by said unmodified nucleic acid sequence;
e) Subjecting the region of step (d) to analysis *in silico* under the same conditions as set out in step (b);
f) Determining whether the at least one nucleotide substitution, insertion, deletion and/or inversion destabilizes the at least one hairpin-like region containing the at least one ribosomal binding site;
g) Optionally repeating steps (d) - (f) as needed until analysis *in silico* under the same conditions as set out in step (b) indicates the destabilization of the at least one hairpin-like region;
h) Preparing the corresponding modified nucleic acid.

A further aspect of the invention provides a modified nucleic acid encoding the p83/30 fragment of MSP-1 of *Plasmodium* having an amino acid sequence as set out in SEQ ID NO: 1, 2 or 3, wherein the modified nucleic acid comprises a nucleotide sequence as set out in any of SEQ ID NOs : 8 or 9.

A further aspect of the invention provides a vector comprising the modified nucleic acid as set out above.

A further aspect of the invention provides a method of preparing the p83/30 fragment of MSP-1 of *Plasmodium,* said method comprising:
a) Providing a modified nucleic acid as set out above and/or a vector as set out above; or preparing a modified nucleic acid by the method as set out above;
b) Incorporating the modified nucleic acid and/or vector of step (a) into a cell, preferably a prokaryotic cell;
c) Incubating the cell of step (b) under conditions allowing expression of the p83/30 subunit of MSP-1; and
d) Recovering the p83/30 fragment of MSP-1.

A further aspect of the invention provides a use of a modified nucleic acid as set out above, or a vector as set out above, in the manufacture of a medicament for preventing and/or treating malaria.

A further aspect of the invention provides a vaccine comprising a modified nucleic acid as set out above, or a vector as set out above.

### Description of the figures

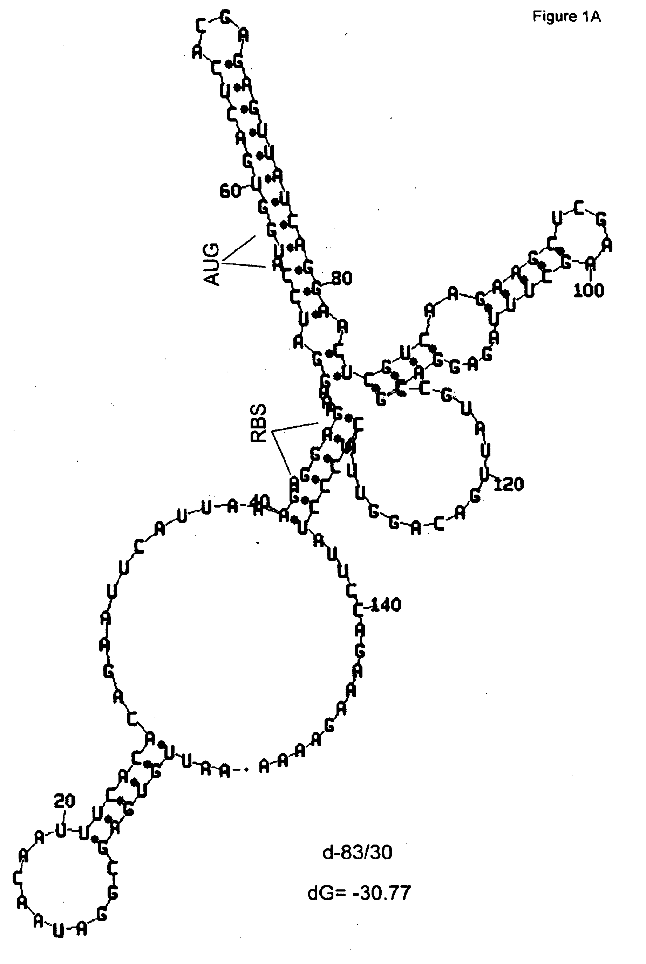

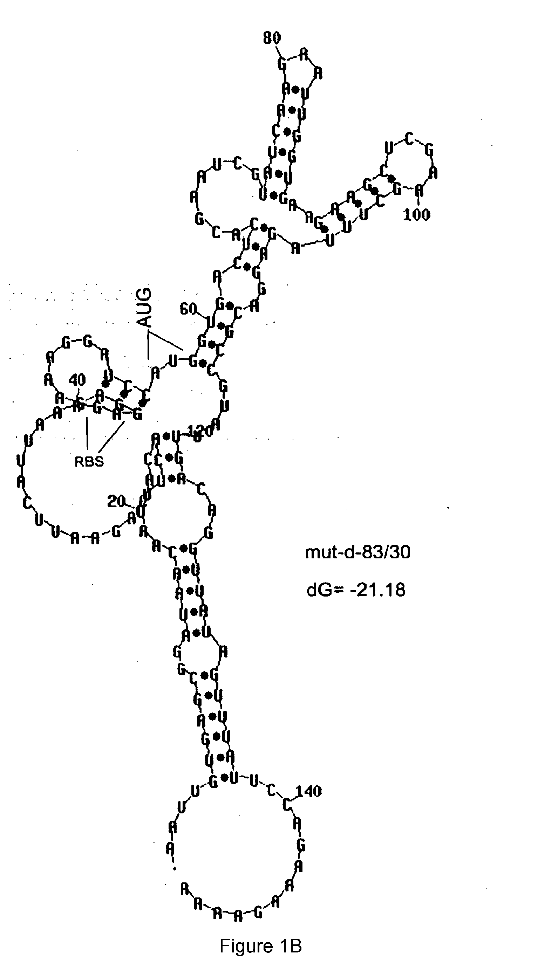

- Fig. 1: Secondary mRNA structures of MSP-1 p83/30 before (A) and after (B) sequence modifications, as predicted *in silico.* The comparison illustrates the marked modification of predicted secondary mRNA structure achieved by destabilizing mRNA secondary structure known or suspected of harboring ribosomal binding sites. The effect of the destabilization was analyzed by *in silico* substitution of single nucleotides. The first 150 bp of the p83/30 mRNA were subjected to *in silico* analysis of secondary structure by using the "mfold' program by Zuker and Turner (version 3.2) provided by the web server of Rensselaer Polytechnic Institute, Troy, NY, USA (for references see hereinbelow). Nucleotides of ribosomal binding site ("RBS") and base-pairing sequences are as indicated. The translation start site (AUG) is also indicated.

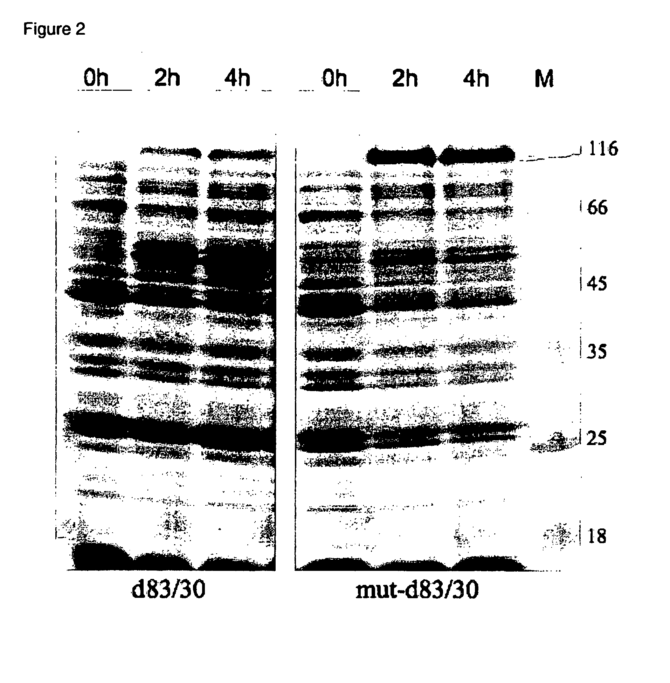

- Fig. 2:: Comparison of the amount of crude protein expression product obtained using unmodified mRNA encoding MSP-1 fragment p83/30 (left, from *Plasmodium falciparum* strain 3D7) and mRNA in which regions of secondary hairpin-like structure containing ribosomal binding sites and/or translation initiation sites have been mutated to destabilize the hairpin-like structure (right) in *E. coli.* MSP-1 protein subunit p83/30 is represented by the dark band in the 2 hour and 4 hour lanes of the gels for both the native and mutated sequences at approximately 116 kD, as measured by the marker ladder at far right. As can be seen by comparing the respective 2 hour and 4 hour lanes in the left and right panels, modification of the MSP-1 p83/30 fragment led to a higher amount of expressed protein (right) than the corresponding unmodified sequence (left).

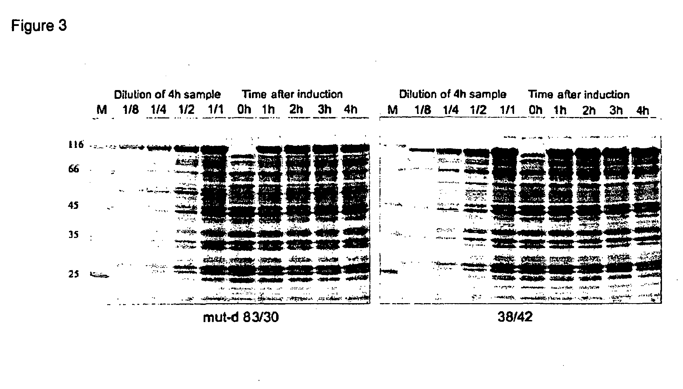

- Fig. 3:: Time course of the production of MSP-1 halves p83/30 (left, based on mutated nucleic acid sequence corresponding to SEQ ID NO: 8 or 9) and p38/42 (right, based on a nucleic acid sequence corresponding to SEQ ID NO: 10). Lanes 1 and 11 (counted from left) show molecular weight marker ladders. Lanes 2-5 and 12-15 (counted from left) show a dilution series for p83/30 and p38/42 fragments of MSP-1, respectively, based on the highest amount of protein obtained after 4 hours of expression. Lanes 6-10 and 16-20 show a time course over 0, 1, 2, 3 and 4 hours of expression for the respective MSP-1 halves p83/30 and p38/42. In each case, the accumulation of protein expression product corresponding approximately to 116 kD corresponds to the desired respective MSP-1 half. 2-fold dilutions up to 1/8 of the original concentration were prepared from the 4h sample. Samples were analyzed by SDS-PAGE and Coomassie staining.

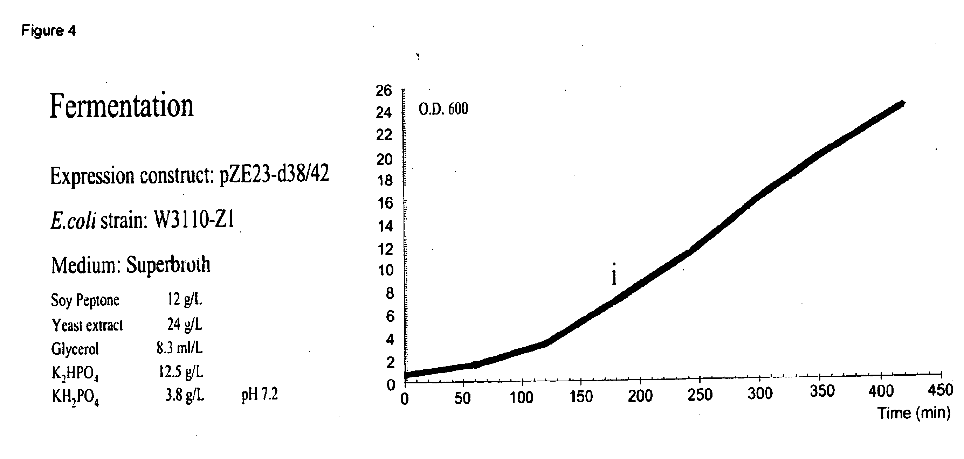

- Fig. 4:: A typical fermentation process in a 15 liter fermenter (Braun, Biostat) shown by example of W3110Z2-p38/42. A typical fermentation process in a 15 liter fermenter is shown. The growth of the culture with time is shown. Induction of expression of the p38/42 fragment of MSP-1 took place at the timepoint indicated by the letter "i" in the figure.

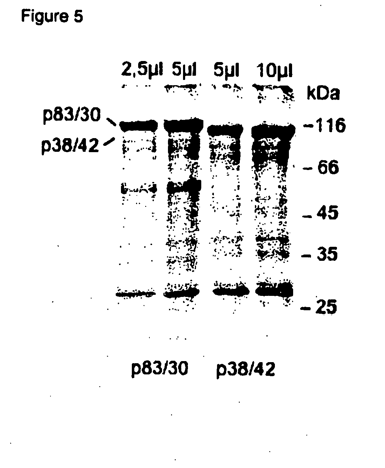

- Fig. 5: Electrophoretic analysis of solubilized IBs. The positions of the two MSP-1 fragments p83/30 and p38/42 are indicated at the side of the gel. SDS-PAGE was performed with 10% polyacrylamide under reducing conditions, followed by Coomassie staining.

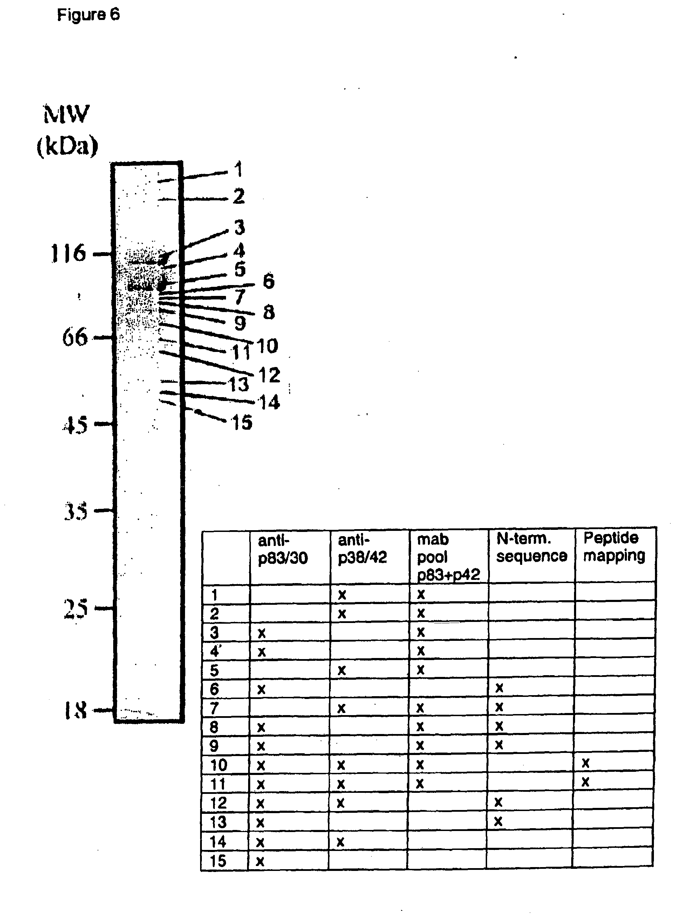

- Fig. 6: Summary of the characterization of proteins present in MSP-1 preparations. The 15 most abundant protein bands are numbered, and were identified by at least one of the following methods (indicated in the title row of the table to the right of the figure): Western Blot with rabbit sera specific for p83/30 ("anti-p83/30), Western Blot with rabbit sera specific for p38/42 ("anti-p38/42), pool of monoclonal antibodies specific for p83 or p42 ("mab pool p83+p42"), direct identification by N-terminal sequencing ("N-term. sequence") and peptide mapping using mass spectrometry ("peptide mapping"). The numbers for the gel bands correspond to the numbers in the leftmost column in the table, with an "x" in the table indicating that a certain analysis method was applied to the protein in the correspondingly numbered band. As can be seen, all detectable components could be identified either by N-terminal sequencing, by peptide mapping via mass spectroscopy or by appropriate monoclonal/polyclonal antibodies against the p83/30 and/or p38/42 fragments of MSP-1.

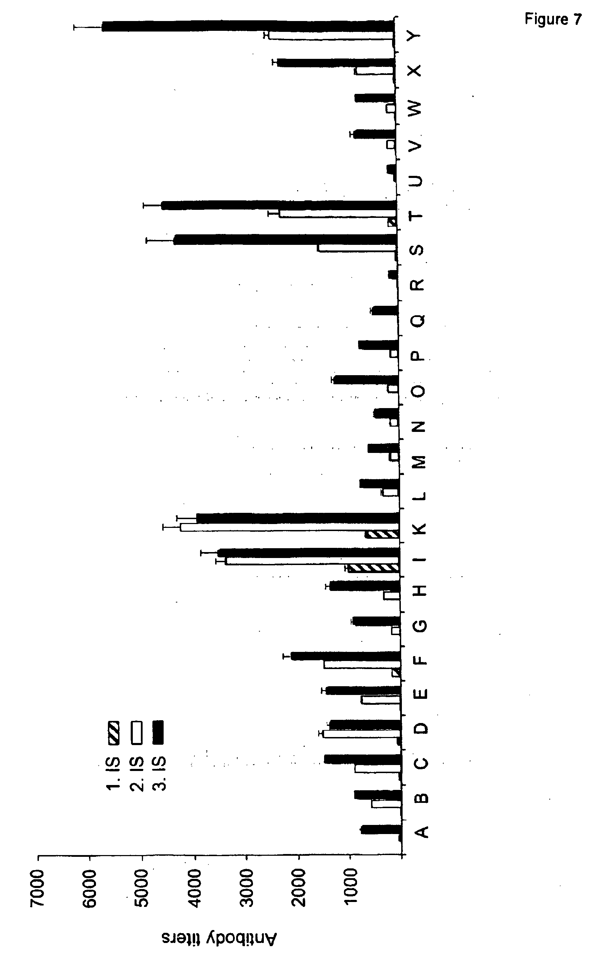

- Fig. 7:: Antibody titers in mouse sera obtained after immunization with MSP-1 in different formulations. Higher bars indicate higher antibody titres, indicating a stronger (and therefore more desirable) immune response to a respective protein. Titers were determined by ELISA using MSP-1 heterodimeric protein made of p83/30 and p38/42 as capture antigen. A: PBS; B: aluminum hydroxide; E: aluminum phosphate; H: MPL (Sigma); I, CpG (Qiagen); L - Q: IC31; R: PBS; S: Montanide ISA720; T: Montanide ISA51: U-W: Virosomes; X, Y: FCA/FIA. IS.1 - IS.3: Sera from first, second or third immunisation.

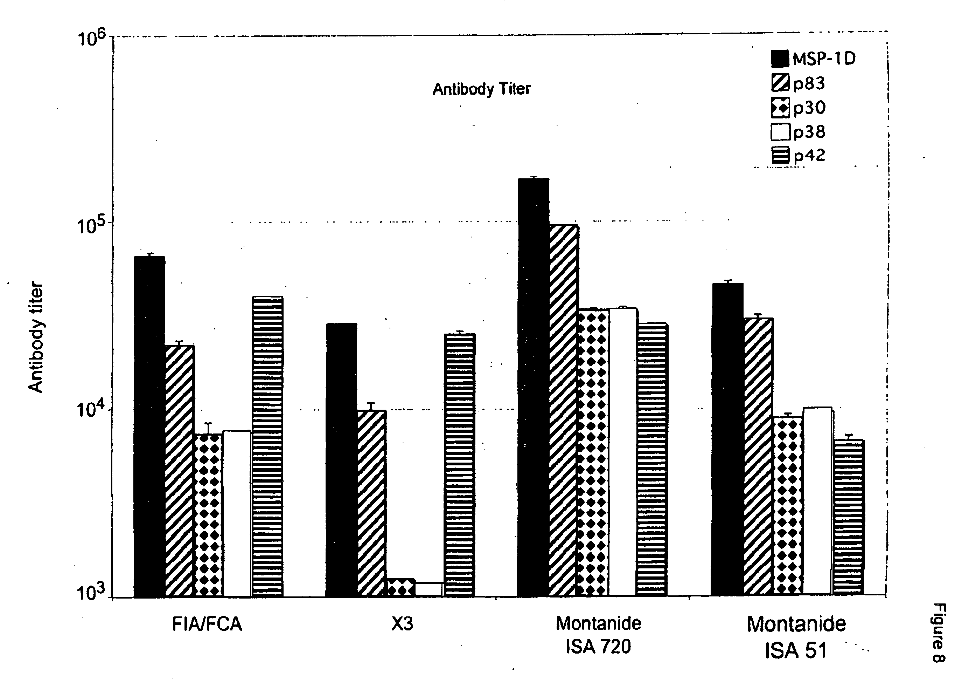

- Fig. 8:: Rabbit antibody titers following immunization with heterodimeric MSP-1 produced as indicated herein (indicated as "MSP-1D" at the upper right of the figure), p83, p30, p38 and p42 together with various adjuvants. Higher bars indicate higher antibody titers, indicating a stronger (and therefore more desirable) immune response to a respective protein. The bars represent results from ELISA studies. Equal amounts of a respective protein as antigen were administered to rabbits (100 µg per injection). The adjuvants used together with the various proteins are indicated below a respective cluster of result bars along the x-axis. The adjuvant "X 3" denotes an adjuvant obtained under a secrecy agreement. As can be seen in the third cluster of result bars, administration of MSP-1 heteromultimer, as well as selected fragments thereof together with montanide, ISA720 led to significantly higher antibody titres in rabbit than observed when same proteins were administered with other adjuvants. This is especially the case for the MSP-1 heterodimer as prepared herein as well as for the individual MSP-1 protein subunits p83, p30 and p38.
- Fig. 9:: Schematic depiction of the primary structure of the MSP-1 derived from *P*. *falciparum* strain 3D7 as "MSP-1 D", a representative of the MAD20 prototype and various fragments thereof which have been recombinantly produced. The numbers below the scheme denote amino acid positions. "SP" denotes "signal peptide. "GA" denotes a glycosynphosphatidylinositol moiety. The arrows at the top of Fig. 9A indicate the sites where the precursor protein is normally cleaved into its major subunits p83 to p42, which are shown in Fig. 9B. A secondary proteolysis cleaves p42 into p33 and p19. The amino acid positions delineate heterologously produced fragments p83, p30, p38 and p42. The isolation tag designations "g", "h" and "s" refer to GST, His₆ and Strep tags, respectively. MSP-1 fragments p83/30 and p38/42, are shown in rows 2 and 4, respectively, of Fig. 9B. In this figure, these fragments bear heterologous isolation tags which can be advantageously omitted under the present invention.

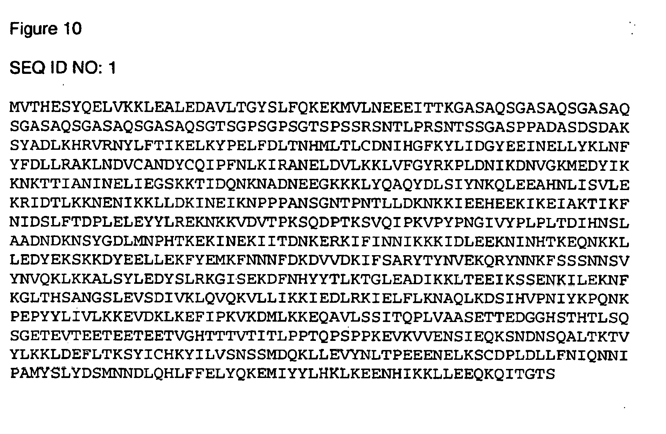

- Fig. 10:: SEQ ID NO: 1. Amino acid sequence of p83/30 from *Plasmodium falciparum* strain 3D7.

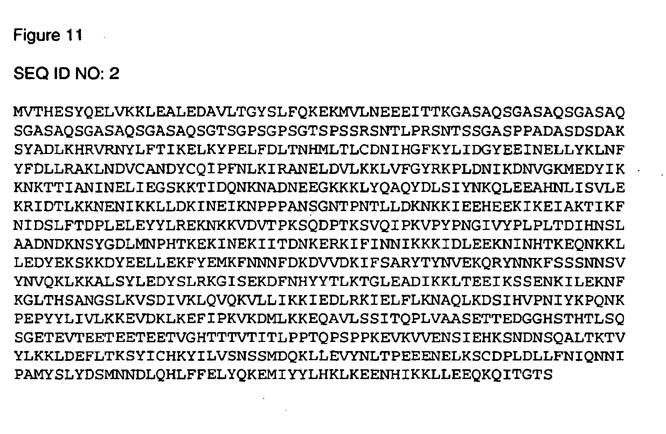

- Fig. 11:: SEQ ID NO: 2. Amino acid sequence of p83/30 from *Plasmodium falciparum* strain 3D7, with two amino acid mutations: E612K and Q767H.

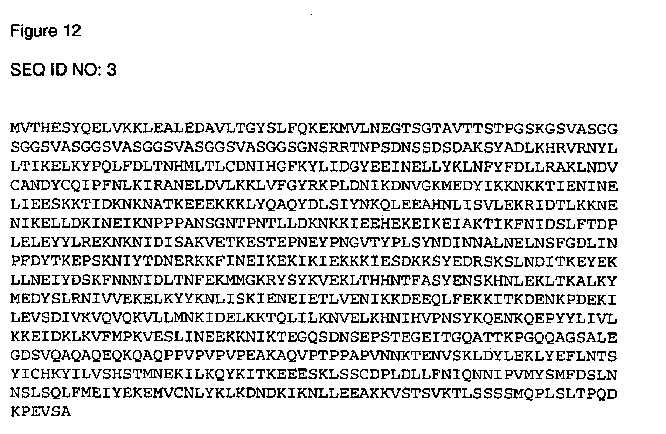

- Fig. 12:: SEQ ID NO: 3. Amino acid sequence of p83/30 from *Plasmodium falciparum* strain FCB-1.

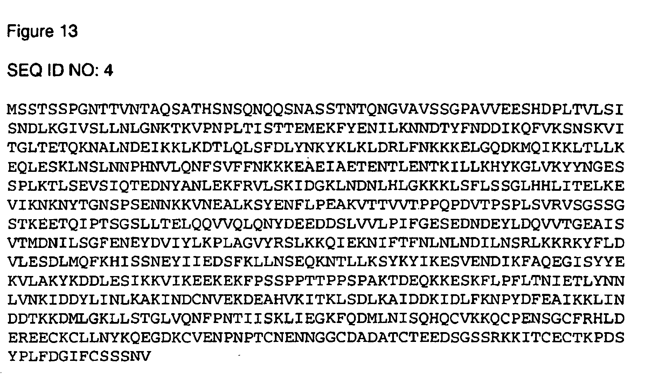

- Fig. 13:: SEQ ID NO: 4. Amino acid sequence of p38/42 from *Plasmodium falciparum* strain 3D7.

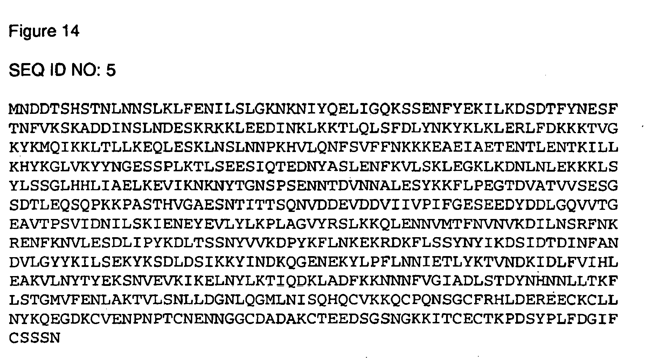

- Fig. 14:: SEQ ID NO: 5. Amino acid sequence of p38/42 from *Plasmodium falciparum* strain FCB-1.

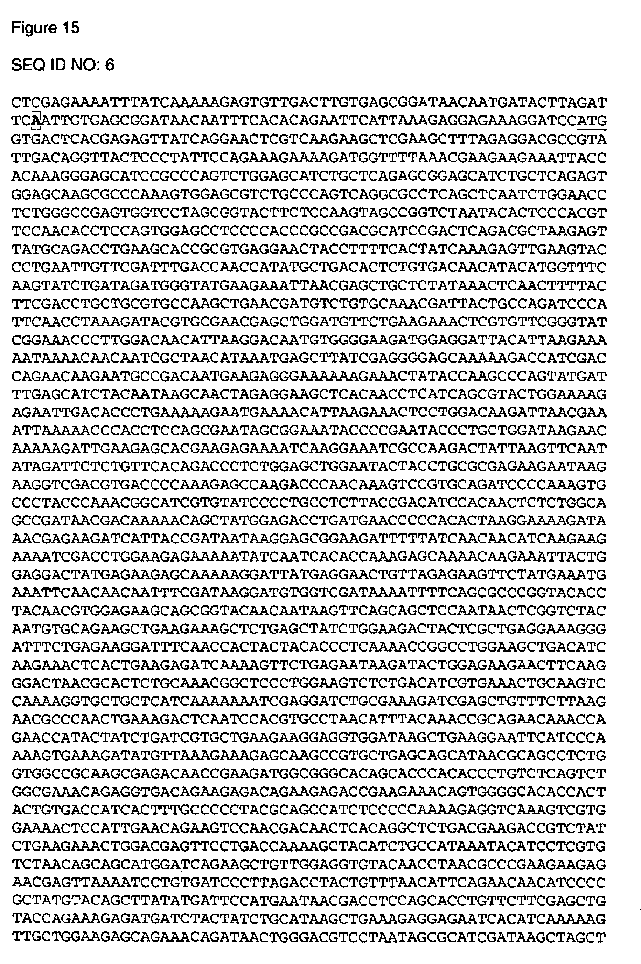

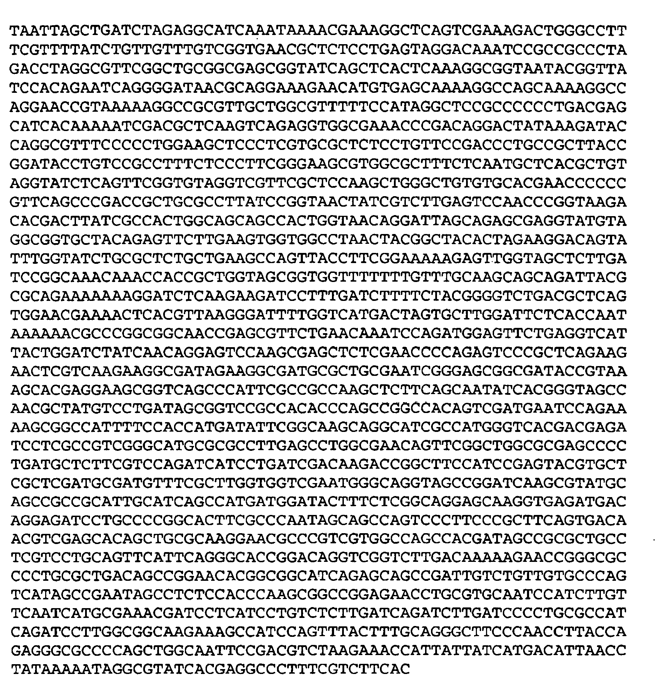

- Fig. 15:: SEQ ID NO: 6. Nucleotide sequence of pZE23/d-83/30 (plasmid encoding p83/30 from *Plasmodium falciparum* strain 3D7 corresponding to SEQ ID NO: 1, i.e. without silent mutations in the RBS and without amino acid mutations E612K and Q767H). The transcriptional start nucleotide A63 is indicated in **shadowed boldface** and the start codon ATG is **underlined.**

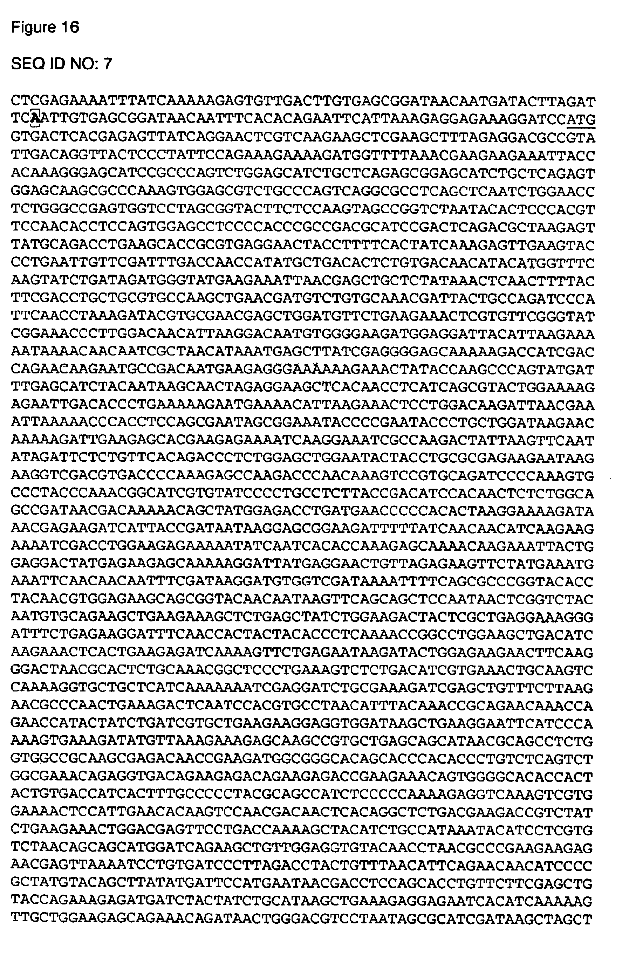

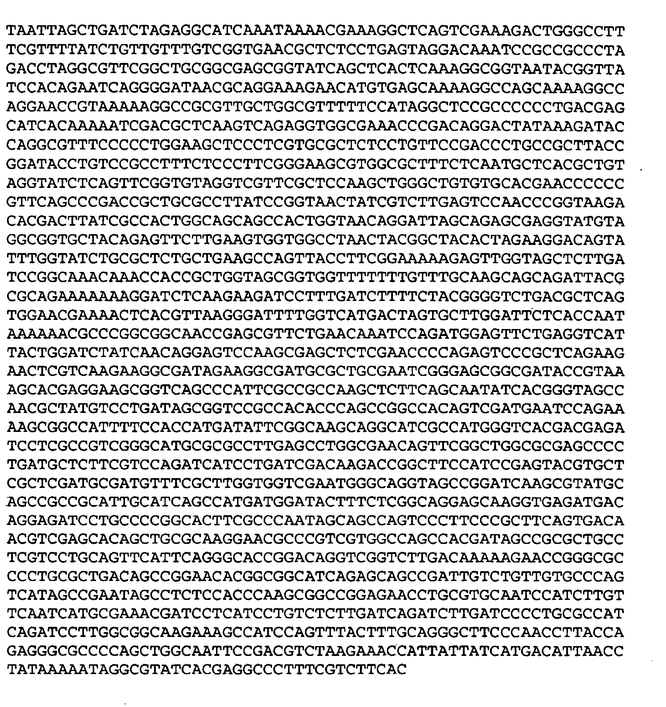

- Fig. 16:: SEQ ID NO: 7. Nucleotide sequence of pZE23/d-83/30 (plasmid encoding p83/30 from *Plasmodium falciparum* strain 3D7 corresponding to SEQ ID NO: 2, i.e. without silent mutations in the RBS but with amino acid mutations E612K and Q767H). The transcriptional start nucleotide A63 is indicated in **shadowed boldface** and the start codon ATG is underlined.

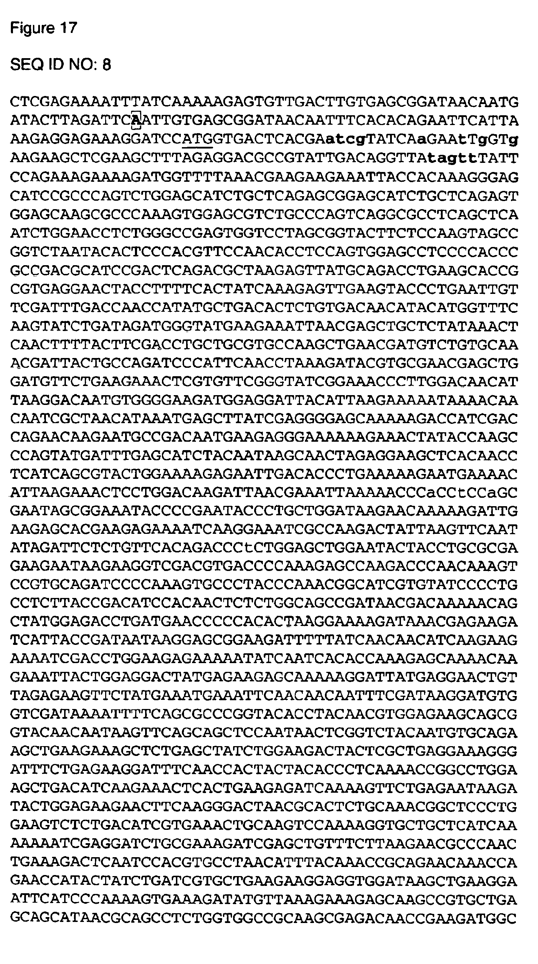

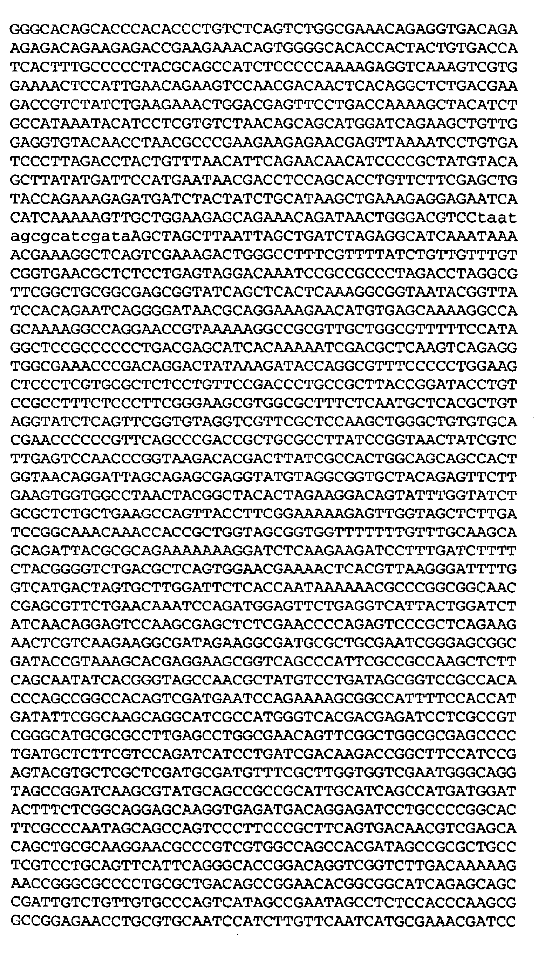

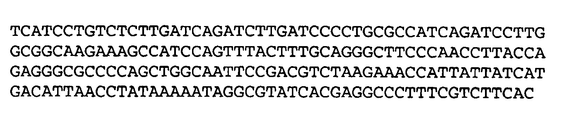

- Fig. 17:: SEQ ID NO: 8. Nucleotide sequence of pZE23/d-83/30 (plasmid encoding p83/30 from *Plasmodium falciparum* strain 3D7 corresponding to SEQ ID NO: 1, i.e. with silent mutations in the RBS but without amino acid mutations E612K and Q767H). The transcriptional start nucleotide A63 is indicated in **shadowed boldface,** the start codon ATG is underlined, and the nucleotide modifications in the region of the ribosomal binding site are in **lower case boldface.**

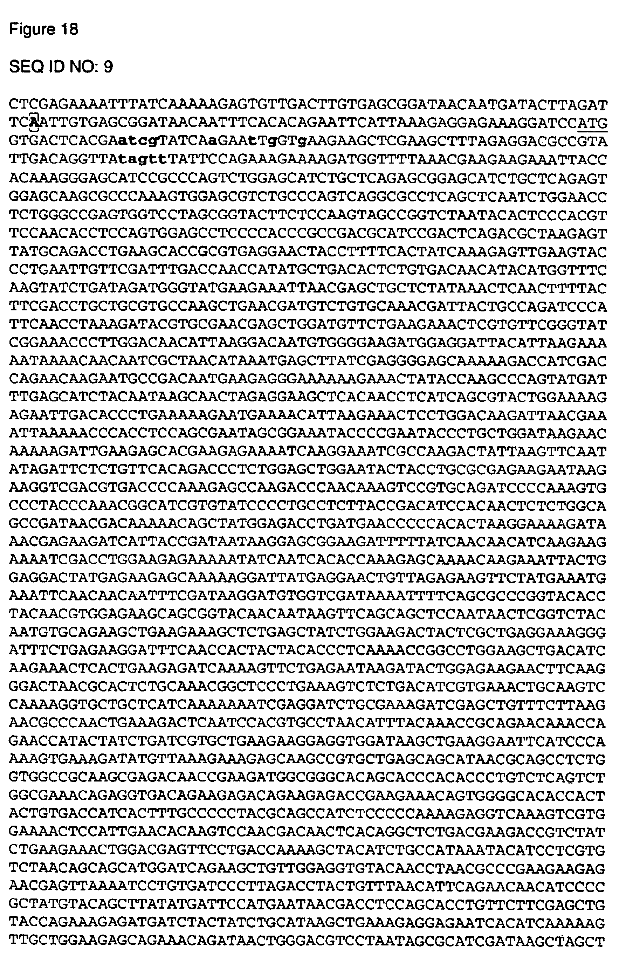

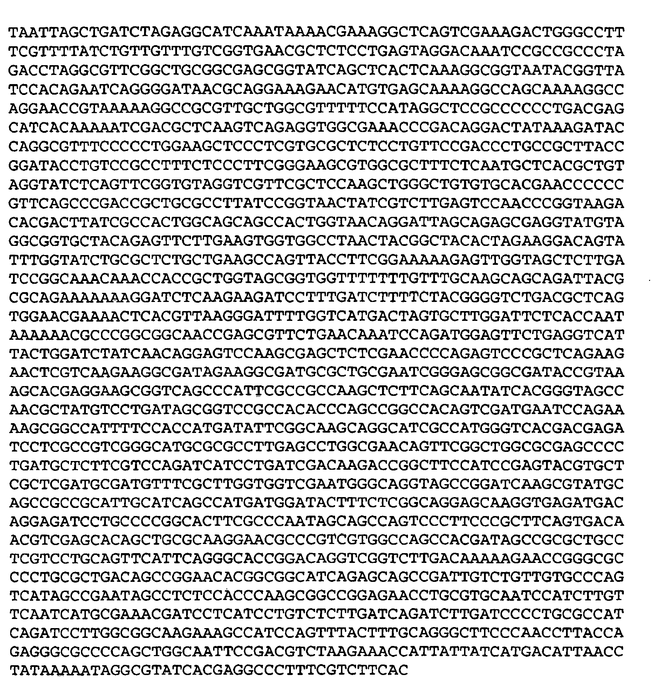

- Fig. 18:: SEQ ID NO: 9. Nucleotide sequence of pZE23/d-83/30 (plasmid encoding p83/30 from *Plasmodium falciparum* strain 3D7 corresponding to SEQ ID NO: 2, i.e. with silent mutations in the RBS and with amino acid mutations E612K and Q767H). The transcriptional start nucleotide A63 is indicated in **shadowed boldface**, the start codon ATG is underlined, and the nucleotide modifications in the region of the ribosomal binding site are in **lower case boldface.**

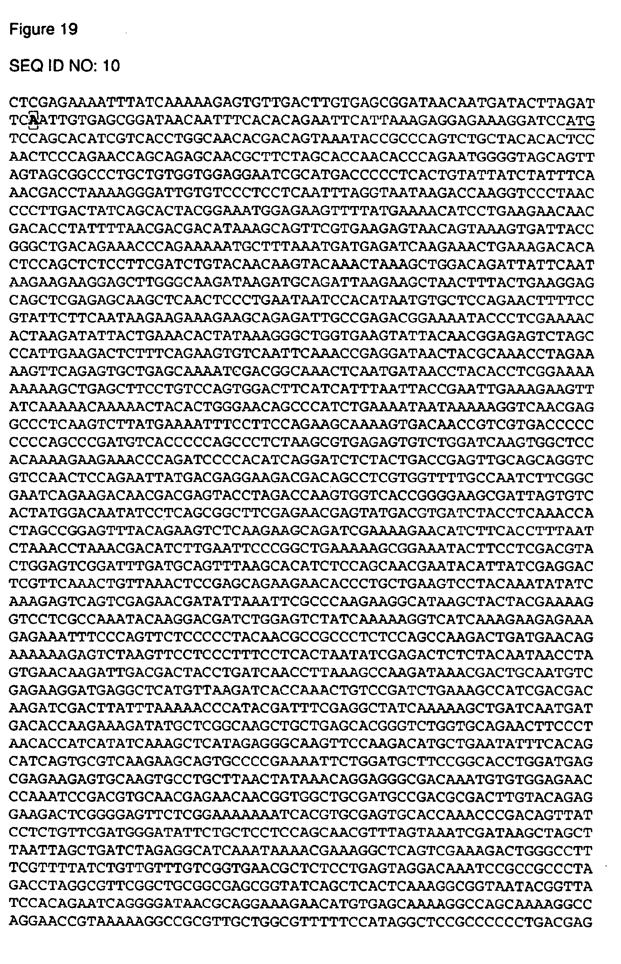

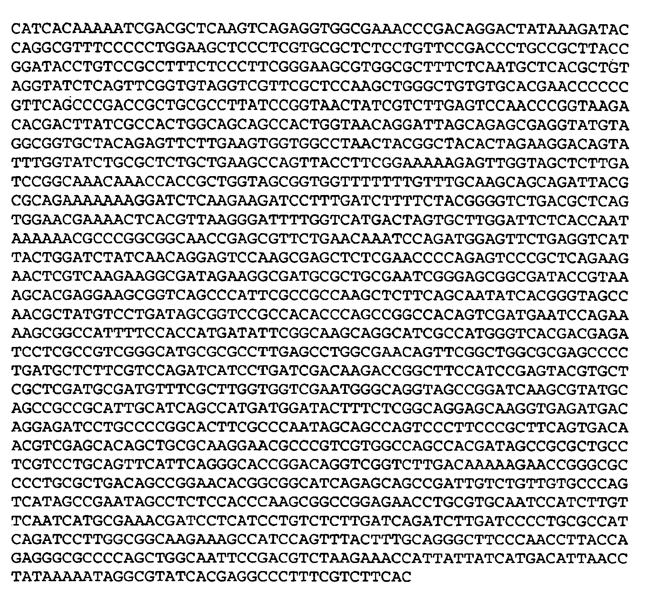

- Fig. 19:: SEQ ID NO: 10. Nucleotide sequence of pZE23/d-38/42 (plasmid encoding p38/42 from *Plasmodium falciparum* strain 3D7 corresponding to SEQ ID NO: 4). The transcriptional start nucleotide A63 is indicated in **shadowed boldface** and the start codon ATG is underlined.

In the context of the present invention, described herein is a merozoite surface protein 1 ("MSP-1") preparation from *Plasmodium,* said MSP-1 preparation comprising
(a) a purified fragment p83/30 of the gp190/MSP-1 from *Plasmodium* without heterologous sequences, and
(b) a purified fragment p38/42 of the gp190/MSP-1 from *Plasmodium* without heterologous sequences.

The MSP-1 preparation is advantageous in that it elicits a strong immune response in host animals, rendering it suitable for use as the active agent in a malaria vaccine. At the same time, the protein is compliant with the Good Manufacturing Practice ("GMP") requirement that therapeutic proteins intended for administration may not contain isolation tags, for example His₆, as such tags interact with/chelate substances in the body of the patient. It should be emphasized that without isolation tags, the MSP-1 would normally have proven difficult to recover at all, let alone in the amounts required for preparation of a vaccine based on this protein. As described hereinbelow, the present inventors have determined how to isolate and recover sufficient amounts of MSP-1 protein as a heterodimer preparation for technical scale preparation of medicaments while complying with regulatory requirements set for therapeutic protein intended for administration to patients. As such, the MSP-1 preparation satisfies an unmet need.

As used herein, the term "MSP-1 preparation" includes MSP-1 comprising two associated, preferably two non-covalently associated protein subunits or fragments ("subunit" and "fragment" being used interchangeably throughout the present application), wherein the two protein subunits are not the same. The term "MSP-1 preparation" thus encompasses heterodimeric MSP-1. For example, the MSP-1 subunits p83/30 and p38/42 are different polypeptides which spontaneously associate non-covalently with one another to form an MSP-1 heterodimer with immunological properties, i.e. epitopes, mirroring those of the native MSP-1 heterotetramer. The term "MSP-1" may also include incomplete portions of a full MSP-1 protein, for example p83/30 or p38/42 "halves" of heterodimeric MSP-1 as well as fragments thereof. In these cases, for purposes of clarity, the respective "half" will generally be referred to as an "MSP-1 fragment" or an "MSP-1 subunit". For purposes of readability, the MSP-1 preparation will be referred to hereinafter simply as "MSP-1", but it is understood that "MSP-1 preparation" as defined above is meant.

As used herein, the term "heterologous sequences" refers to sequences which are not MSP-1 sequences. For example, a part of a polypeptide, for example an extension of a desired polypeptide at the N- and/or C-terminal end, comprising a region of specific sequence intended to specifically interact with a predetermined binding partner may be considered a "heterologous sequence". Such "isolation tags" are commonly applied in protein chemistry to aid in the isolation/recovery of recombinantly produced proteins. Examples of commonly used heterologous tags include His₆ (which chelates divalent nickel), glutathione S-transferase ("GST", which binds glutathione), and "strep" (8-amino acid tag which binds to engineered streptavidin). As used herein, the term "heterologous sequence" also refers to any chemical modification of an expressed protein which is effected with the purpose of enabling protein recovery by selective binding methods. The above MSP-1 comprises no such heterologous sequences.

As used herein, the term "purified fragment" (referring to p83/30) refers to a single polypeptide comprising the p83 portion and the p30 portion of MSP-1 of *Plasmodium.* Preferably, MSP-1 protein comprises a p83/30 fragment from a *Plasmodium* F-strain or a *Plasmodium* D-strain. The purified p38/42 fragment is a single polypeptide comprising the p38 portion and the p42 fragment of MSP-1 from *Plasmodium.* The p38/42 fragment may be derived from any *Plasmodium* strain, e.g. a D-strain or an F-strain of *Plasmodium falciparum.*

As used herein, the terms "p83/30 fragment" and "p38/42 fragment" also refer to modified fragments which have an amino acid sequence identity of at least 90%, preferably of at least 95% and more preferably of at least 98% over the entire length of the polypeptide to a native p83/30 or p38/42 fragment. The terms also encompass truncated fragments which may comprise deletions of single amino acids and/or amino acid portions of up to 10, more preferably up to 5 amino acids compared to the wild-type polypeptide. A p83/30 fragment preferably has a length of at least 800 amino acids, more preferably of at least 850 amino acids. Further, the p83/30 fragment has a sequence identity of at least 90%, more preferably at least 95% and, most preferably, at least 98% with the p83/30 fragment of the *P. falciparum* F-variant in SEQ ID NO: 3 or the *P. falciparum* D-variants of either SEQ ID NO: 1 or 2.

The MSP-1 preparation as well as compositions comprising the MSP-1 preparation advantageously do not comprise any fragments or other polypeptide products not deriving from the MSP-1 fragments p83, p30, p38 or p42.

According to one embodiment, the heterologous sequence is an isolation tag.

According to a further embodiment, the purified p83/30 fragment is an F-fragment, i.e. a fragment derived from an F-strain of *Plasmodium,* particularly from *Plasmodium falciparum* strain FCB-1 also known as FC or F. It has been found that the F-fragment is more stable against proteolytic degradation than the D-fragment, i.e. a fragment derived from a D-strain of *Plasmodium,* particularly from the *Plasmodium falciparum* strain 3D7 known as NF54. It has also been found that the p83/30 fragment may be combined with a heterologous or a homologous p38/42 fragment, for example with a heterologous p38/42 D-fragment and/or a p38/42 F-fragment.

Components a) and b) are preferably recombinant polypeptides, i.e. polypeptides which have been manufactured in a recombinant host cell, e.g. a eukaryotic host cell such as a yeast cell, e.g. *S. cerevisiae* or *P*. *pastoris,* or in prokaryotic cells, e.g. gram-negative bacterial cells such as E. coli. Preferably, the recombinant host cell is an E. coli cell. More preferably, the host cell is *E. coli* W3110Z2, for example E. coli W3110Z2-pZE23/d-83/30 (DSM accession number 16602) or W3110Z2-pZE23/d-38/42 (DSM accession number 16600). The DSM numbers represent those assigned by the *Deutsche Sammlung von Mikroorganismen und Zellkulturen* (German Collection of Microorganisms and Cell Cultures), in Braunschweig, Germany.

In one preferred embodiment, the p83/30 fragment preferably comprises the amino acid sequence of an F-strain of *P*. *falciparum* as shown in SEQ ID NO: 3 and optionally an N-terminal signal peptide sequence or a modified F-fragment derived from the F-strain sequence. Alternatively, the p83/30 fragment comprises the amino acid sequence of a D-strain of *P*. *falciparum* and optionally an N-terminal signal peptide sequence of a modified D-strain sequence. The amino acid sequence shown in SEQ ID NO: 2 is derived from the amino acid sequence of a D-strain and comprises 2 amino acid substitutions at positions 612 (E → K) and 767 (Q → H). As a further alternative, the p83/30 fragment comprises the amino acid sequence of a D-strain of *P. falciparum* as shown in SEQ ID NO: 1 and optionally an N-terminal signal peptide sequence of a modified D-strain sequence.

The p38/42 fragment may be derived from an F-strain of *P*. *falciparum* as shown e.g. in SEQ ID NO: 5 and/or from a D-strain of *P. falciparum* as shown in SEQ ID NO: 4. It was surprisingly found that a p83/30 fragment of an F-strain may be combined with both a homologous p38/42 fragment from an F-strain or with a heterologous p38/42 fragment from a different *P. falciparum* strain, e.g. a D-strain.

The p38/42 fragment preferably has a length of at least 700 amino acids and more preferably at least 750 amino acids. Further, it is preferred that the p38/42 fragment has a sequence identity of at least 90%, more preferably at least 95% and, most preferably, at least 98%, compared to the p38/42 fragments from a *P. falciparum* F-strain (SEQ ID NO: 5) and/or from a D-strain (SEQ ID NO: 4).

The amino acid sequences of particularly preferred embodiments of p83/30 and p38/42 are shown in SEQ ID NO: 1-5, and preferred combinations of the p83/30 fragment of MSP-1 with the p38/42 fragment of MSP-1 to form heterodimeric MSP-1 are thus as set out in the following Table 1:

**Table 1**

| **p83/30 fragment** | **p38/42 fragment** |
|---|---|
| SEQ ID NO: 1 | SEQ ID NO: 4 |
| SEQ ID NO: 1 | SEQ ID NO: 5 |
| SEQ ID NO: 2 | SEQ ID NO: 4 |
| SEQ ID NO: 2 | SEQ ID NO: 5 |
| SEQ ID NO: 3 | SEQ ID NO: 4 |
| SEQ ID NO: 3 | SEG ID NO: 5 |

In the present invention, it is preferred that the *Plasmodium* species is *Plasmodium falciparum.*
In one aspect the present invention provides a method for preparing a modified nucleic acid encoding the p83/30 fragment of MSP-1 from *Plasmodium* having an amino acid sequence as set out in SEQ ID NO: 1, 2 or 3, said method comprising:
a) Providing an mRNA sequence encoding the p83/30 subunit of MSP-1 from *Plasmodium* having an amino acid sequence as set out in SEQ ID NO: 1, 2 or 3;
b) Subjecting the region surrounding the start codon of the mRNA sequence of step (a) to analysis *in silico* to determine the predicted secondary structure of said mRNA sequence;
c) Identifying at least one stable, hairpin-like structure in said region surrounding the start codon of step (b);
d) Modifying the sequence of the mRNA in said region surrounding the start codon of step (b), wherein at least one nucleotide substitution, insertion, deletion and/or inversion destabilizes said hairpin-like region, and wherein the at least one nucleotide substitution, insertion, deletion and/or inversion is effected *in silico* so as to encode the same amino acid sequence as encoded by said unmodified nucleic acid sequence;
e) Subjecting the region of step (d) to analysis *in silico* under the same conditions as set out in step (b);
f) Determining whether the at least one nucleotide substitution, insertion, deletion and/or inversion destabilizes the at least one hairpin-like region containing the at least one ribosomal binding site;
g) Optionally repeating steps (d) - (f) as needed until analysis *in silico* under the same conditions as set out in step (b) indicates the destabilization of the at least one hairpin-like region;
h) Preparing the corresponding modified nucleic acid.

Previously, preparation of sufficient quantities of heterodimeric MSP-1 comprising the MSP-1 protein subunits p83/30 and p38/42 has been limited by the low expression of the p83/30 subunit. The present inventors have effectively removed this bottleneck in the preparation of MSP-1 comprising the p83/30 and p38/42 subunits by determining and eliminating the source of the low expression observed. Specifically, the inventors have determined that the region surrounding the start codon in the unmodified mRNA encoding the p83/30 subunit of MSP-1 (e.g. with amino acid sequence as set out in SEQ ID NO: 1, 2 or 3) contains multiple regions of secondary structure, for example hairpin-like regions, which impede recognition by the ribosomal machinery required for translation. By determining which regions of the mRNA exist in stable secondary structure, for example hairpin-like structures, the inventors have determined that targeted modifications to these regions of the nucleic acid sequence can be effected so as to destabilize the secondary structure and render previously inaccessible regions of mRNA available for recognition by ribosomes. Since protein expression levels depend in great part on proper ribosomal recognition of its binding sites on mRNA, rendering these sites more accessible to the ribosome leads to greatly increased expression levels of the corresponding protein, thus eliminating the previously limiting step in the recombinant production of MSP-1.

As used herein in this and other aspects/embodiments, the term "modified" refers to the fact that at least one change has been made in the coding nucleotide sequence of a starting nucleic acid, said change resulting in the destabilization of the determined or suspected regions of secondary hairpin-like structure in the corresponding mRNA of this nucleic acid. However, a modified nucleic acid need not be mRNA as such. While the term "modified nucleic acid" includes mRNA, it also encompasses a DNA sequence which, when transcribed into mRNA, will have no or at least reduced or different secondary hairpin-like structure in the regions corresponding to ribosomal binding sites, as compared to the corresponding nucleic acid sequence prior to modification. Further, when the modification effected is within the ORF encoding the p83/30 MSP-1 protein subunit, the modification preferably results in a nucleotide triplet which, due to the degeneracy of the genetic code, is translated as the same amino acid as would result from translation of the corresponding triplet in unmodified form. This is referred to in this and other aspects/embodiments as a "silent modification" or a "silent mutation".

As used herein in this and other aspects/embodiments, the term "region surrounding the start codon" refers to a window of nucleotides starting at the transcriptional start site, and extending over the AUG start codon about 100-150 nucleotides into the coding region, to comprise a total window length of about 150-200 nucleotides.

As used herein in the present and other aspects, the term "about" typically denotes variance of up to and including ±15% (explicitly including all intermediate values of whole or decimal value between 0-15%), the result of this variance being rounded up or down to the nearest whole number as appropriate.

As used herein in this and other aspects/embodiments, the term "ribosomal binding site" refers to stretches of nucleotides known to be bound by ribosomes as part of the process of translating mRNA into an amino acid sequence. The skilled person understands how to recognize these sequences. For example, in prokaryotes the ribosomal binding site, also referred to as the "Shine-Dalgarno sequence" or the "Shine-Dalgarno box" (after Shine & Dalgarno (1975) Nature 254, 34-8) consists of purine-rich sequences approximately 10 nucleotides upstream of the start codon. The ribosomal binding site sequence used for the expression of the p83/30 fragment of MSP-1 under the present invention corresponds to the Shine-Dalgarno consensus sequence, the ability of which to drive gene expression in E. coli has been previously described (Lutz & Bujard (1997) Nucleic Acids Res. 25, 1203-10

As used herein in this and other aspects/embodiments, the term *"in silico"* refers to analysis done electronically, for example on a computer using appropriate software for the analysis of nucleic acids and their predicted secondary structure. Suitable software platforms for nucleic acid sequence analysis are known to the skilled person, for example the "mfold" program by Zuker and Turner (version 3.2) provided by the web server of Rensselaer Polytechnic Institute, Troy, NY, USA (Zuker (2003) Nucleic Acids Res. 31 (13), 3406-15; Mathews et al. (1999) J. Mol. Biol. 288, 911-40). Alternative sources are the "Vienna RNA package" (available on the internet under: http://www.tbi.univie.ac.at/~ivo/RNA/) or "RNA world" at IMB Jena (available on the internet under: http://www.imb-jena.de/RNA.html). The prediction programs used by these servers use the same strategy for structure prediction based on an algorithm for calculating the free minimal energy of polyribonucleotide molecules (see: Stiegler & Zuker (1981) Nucleic Acids Res. 9, 133-48). While the different online resources may apply different optimization packages of this algorithm, analysis results obtained using the mRNA sequence of an MSP-1 p83/30 fragment subjected tc structure prediction by different servers did not deviate from one another significantly with respect to the predicted secondary structure of the RBS. Thus, any of these programs/algorithims may be equivalently used with comparable results. In the online resources mentioned above, default values may be used for the prediction of RNA secondary structure. These default values are designed to reflect conditions in vivo. In contrast to parameters for the prediction of secondary structure in DNA, parameters for RNA folding cannot be adjusted with the user interface of the "mfold" server.

As used herein in this and other aspects/embodiments, the term "destabilize" or "destabilization" and the like refer to a decrease in the stability, and therefore persistence of, secondary structure such as hairpin-like structure in mRNA as compared to the persistence of secondary structure in the same region when no nucleotide modifications have been carried out. For example, modifying the nucleic acid sequence such that hybridization between two respective nucleotides or nucleotide stretches is disrupted and would be less likely to occur in a modified state as compared to an unmodified state would constitute a "destabilizing". As such, "destabilization" is not just the result of a lower free energy of the predicted molecule. Rather, it focuses on the secondary structure of the ribosomal binding site, with any involvement in a defined secondary structure being more "stable" than an unstructured region. For example, and while not being bound by theory, the present inventors postulate that part of a ribosomal binding site which, in an unmodified sequence, participates in a hairpin-like structure will be less accessible for binding by the ribosome than it will be in a sequence modified so as to reduce the degree of defined structure in this region.

As used herein in this and other aspects/embodiments, the term "hairpin-like region" denotes a region in which a first subregion of mRNA in the 5' → 3' direction forms a stably hybridized structure with a second subregion of mRNA in the 3' → 5 direction, where the first and second subregions each form part of the same contiguous strand of mRNA. In a typical "hairpin" or "stem-loop" structure, very few nucleotides, typically 5-6 exist between the hybridized first and second subregions participating in the hairpin. In contrast, there is no particular limit to the number of nucleotides which may exist between the first and second hybridized subregions of the "hairpin-like region" of the coding mRNA for the MSP-1 p83/30 fragment. As can be seen in appended Fig.1, very many nucleotides may exist between the first and second subregions of the mRNA participating in the hairpin-like region.

In the context of the present invention, described herein is a composition comprising the above MSP-1, said MSP-1 comprising
a) a purified fragment p83/30 of the gp190/MSP-1 from *Plasmodium* without heterologous sequences, and
b) a purified fragment p38/42 of the gp190/MSP-1 from *Plasmodium* without heterologous sequences.

According to one embodiment, the components (a) and (b) in the composition are preferably present in equimolar amounts, e.g. molar ratios of from 1.5:1 to 1:1.5, more preferably from 1.2:1 to 1:1.2 and most preferably from about 1:1 of component (a) to component (b). Preferably, at least 70%, more preferably at least 80% and most preferably at least 90% of the fragments in the composition are present as non-covalently associated dimer.

In a preferred embodiment, the composition has a purity of at least 95% and, more preferably, of at least 97.5%, as determined by SDS gel electrophoresis and silver staining. As used herein, the term "purity" refers to the absence of heterologous polypeptides, i.e. non-MSP-1 polypeptides, e.g. isolation tags.

In a further preferred embodiment, the composition has a content of degradation products of less than 30%, more preferably of less than 20% and most preferably of less than 15%, as measured by SDS gel electrophoresis and immuno-staining. In this context, the term "degradation products" refers to polypeptide molecules which result from a degradation of the p83/30 fragment or the p38/42 fragment as described above.

In the content of the present invention, described herein is a pharmaceutical composition comprising the above MSP-1, said MSP-1 comprising
a) a purified fragment p83/30 of the gp190/MSP-1 from *Plasmodium* without heterologous sequences, and
b) a purified fragment p38/42 of the gp190/MSP-1 from *Plasmodium* without heterologous sequences.

In one embodiment the pharmaceutical composition may additionally comprise pharmaceutically acceptable carriers, diluents and/or adjuvants.
A further aspect of the invention provides a modified nucleic acid encoding the p83/30 subunit of MSP-1 from *Plasmodium* having an amino acid sequence as set out in SEQ ID NO: 1, 2 or 3, wherein the modified nucleic acid comprises a nucleotide sequence as set out in any of SEQ ID NOs : 8 or 9 A further aspect of the invention provides a vector comprising the modified nucleic acid as set out above.

In one embodiment, the vector is a polynucleotide vector, for example a plasmid, for example a plasmid of the pZ series (Lutz & Bujard (1999) Nucleic Acids Res. 25, 1203-10). Other suitable plasmid vectors are known to one of ordinary skill in the art. It is preferred that the polynucleotide vector further comprises a promoter operably linked to the modified nucleic acid encoding the p83/30 fragment of MSP-1 so as to drive transcription thereof. In another embodiment the polynucleotide vector may comprise an inducible promoter such as P_{A1lacO-1} .

In another embodiment, the vector is a viral vector, for example a vaccinia viral vector, for example modified vaccinia vector Ankara (MVA), an adenovirus such as adenovirus serotype 5, or a measles virus such as an attenuated strain from Edmonton isolate. As is well known to the skilled person, viral vectors such as MVA are suitable for manufacturing safe genetic vaccines in which a nucleic acid or nucleic acids encoding an antigenic polypeptide(s) intended to immunize a subject is/are introduced into the body of subject, where the antigenic polypeptides are then expressed, recognized by the subject's immune system, and lead to the mounting of an innate and/or adaptive immune response against the expressed polypeptide(s). Alternatively, the vector may for example be attenuated Mycobacterium tuberculosis.
A further aspect of the invention provides a method of preparing the p83/30 fragment of *Plasmodium* MSP-1, said method comprising:
a) Providing a modified nucleic acid as set out above and/or a vector as set out above; or preparing a modified nucleic acid by the method as set out above.
b) Incorporating the modified nucleic acid and/or vector of step (a) into a cell, preferably a prokaryotic cell;
c) Incubating the cell of step (b) under conditions allowing expression of the p83/30 fragment of *Plasmodium* MSP-1; and
d) Recovering the p83/30 fragment of *Plasmodium* MSP-1.

The inventors have determined that using a p83/30-encoding nucleic acid sequence modified as described hereinabove (e.g. as set out in SEQ ID NO: 8 or 9) leads to greatly increased expression of the p83/30 fragment of *Plasmodium* MSP-1, allowing very efficient production of this protein fragment within the host cell. Without being bound by theory, the inventors believe that the rapid increase in protein levels in a host transfected with the modified nucleic acid sequence as set out above leads to rapid aggregation and precipitation of this protein as inclusion bodies before the translated protein can be subjected to intracellular proteolytic degradation. Accordingly, it is believed that the p83/30 protein produced is rapidly converted into inclusion body form, where the protein remains protected from degradation by endogenous proteolytic enzymes of the host cell.

Without being bound by theory, the inventors thus believe that the amounts of p83/30 fragment protein obtained upon recovery are greater than previously possible for two reasons: First, the use of modified nucleic acid as set out above (e.g. SEQ ID NO: 8 or 9) enables more efficient recognition by ribosomes and initiation of translation than possible for unmodified nucleic acid, where ribosomal recognition sites remain buried in regions of secondary structure. This enables translation to begin more efficiently. Second, once translation of the p83/30 subunit is complete, this protein appears to be converted rapidly to inclusion bodies, where it remains protected from degradation by the host cell until it is recovered.

As used herein in this and other aspects/embodiments, the term "conditions allowing expression" denote any environmental conditions known to the skilled person to growth and/or promote protein expression from a chosen host expression system, i.e. a chosen strain of host cell. These conditions may include for example, temperature, light, shaking speed, pH of the medium, nutrient composition of the medium as well as the presence or absence of growth factors, amino acids, vitamins and the like known to promote protein expression in the type of host cell chosen. For example, using E. *coli* as a host cell for the heterologous preparation of the p83/30 fragment of *Plasmodium* MSP-1, suitable conditions may be as set out in the appended Examples, e.g. Example 3.

In one embodiment, the recovering step comprises solubilizing the MSP-1 protein subunit from inclusion bodies formed within the host cell. As used herein in this and other aspects/embodiments, the term "inclusion bodies" refers to protein aggregates in the cytoplasm of bacteria that are formed by heterologously expressed genes. For many recombinant proteins the environment in the prokaryotic cytoplasm is not suitable for proper protein folding, particularly when the protein is present at high concentration due to overexpression. The proteins precipitate and form large protein aggregates termed "inclusion bodies" that can be identified microscopically as high molecular weight structures inside the bacteria. Protein in the inclusion bodies is usually not present in its natural conformation and must therefore be refolded *in vitro* from inclusion bodies before it can function properly. As the information for refolding is contained in the amino acid sequence of a protein, refolding procedures differ for each protein and need to be established in detail on a case-by-case basis, as dictated by the particular protein expressed and present as inclusion bodies. Nevertheless, certain basic parameters have been identified which are advantageous for the folding of a wide variety of proteins (see for example Rudolph et al. (1997) Protein function: A practical approach. Ed. T.E. Creighton). One of ordinary skill in the art understands how to apply these general parameters (e.g. choice of renaturation buffers) to the isolation and refolding of protein in the form of inclusion bodies.

The inventors have found that one particularly efficient method for renaturing solubilized inclusion bodies of MSP-1 proteins (both p83/30 and p38/42 fragments thereof) involves pulsing the solution containing the solubilized inclusion bodies. In this sense, "pulsing" refers to the addition of the respective protein solution into a renaturation buffer in which refolding is to take place. This process is termed "pulse renaturation" and has been described previously (Rudolph & Lillie (1996) FASEB J. 10, 49-56). Briefly, small amounts of denatured proteins are added (i.e. "pulsed") into a renaturation buffer in separate aliquots, spaced by time intervals, e.g. at least one hour. During a respective interval, protein folding can occur at low concentrations, thereby decreasing the probability of aggregation. For example, instead of adding 1 mg of protein to the renaturation solution all at once, it can be advantageous to add the protein gradually, in smaller amounts or "pulses", for example stirring shortly before, during and shortly following a respective pulse. In this way, for example, 10 individual additions of 0.1 mg of protein each may be added over, for example, 10 hours; one pulse per hour briefly preceded and followed by stirring, and with the solution being allowed to incubate at rest prior to the next respective addition of protein. Refolded protein does not interfere with the renaturation of additional protein which has not yet refolded.

In the renaturation of p83/30 and p38/42 fragments of *Plasmodium* MSP-1, the inventors have found it particularly advantageous to add protein in intervals of about one hour, a time span which has been previously shown to allow a wide variety of proteins to refold. For the application, or "pulsing", of protein, any device allowing the addition of a defined volume of solution may be used, e.g. automated pipettor. The inventors have found it to be particularly advantageous to stir the solution about one minute before, during and about one minute after a respective protein addition. These procedure settings, as well as the approximate one hour time interval between respective protein additions, have been previously described (Rudolph & Lillie (1996) FASEB J. 10, 49-56).

Such pulse-renaturation techniques are especially applicable to large scale preparatory processes of the type needed for preparing the large amounts of MSP-1 subunits required for the manufacture of vaccines. Further, in the event that host cells are transfected with nucleic acid encoding only the p83/30 MSP-1 protein subunit, then the inclusion bodies should contain only this protein, meaning that it can be efficiently isolated from the cell lysate by standard physical separation techniques (such as centrifugation). The high-yield, inclusion body-forming process developed and implemented by the inventors thus allows efficient isolation of the p83/30 MSP-1 protein subunit in a way which completely avoids any dependence on isolation tags.

In the context of the present invention, described herein is a p83/30 subunit of the MSP-1 protein obtainable by the corresponding preparation method set out above. As explained above, previous attempts to prepare amounts of MSP-1 protein fragment p83/30 sufficient for technical scale preparation of a vaccine failed due to a) low heterologous expression levels and b) proteolytic degradation by the host cell of the small amounts expressed. The vastly improved yields of MSP-1 subunit p83/30 are the result of the inventors' recognition of which types of modifications need to be effected in nucleic acid encoding this protein subunit. These modifications allow more efficient, rapid expression of the p83/30 protein product. The rapid production of this protein in host cells leads to the accumulation of inclusion bodies, in which form the p83/30 subunit remains protected from degradation by the host cell. Since the p83/30 subunit is present in large quantities in easily separable inclusion bodies, it is isolable in highly pure form using standard physical separation techniques such as centrifugation, avoiding the need to rely on isolation tags for protein recovery.

The inventors' recognition of the need for specific expression-enhancing modifications in the nucleic acid encoding the p83/30 subunit of MSP-1 thus in effect eliminates the one step which has hitherto hampered technical scale preparation of MSP-1 heterodimers comprising this subunit. At the same time, GMP-compliant protein is obtained which can be submitted to the appropriate regulatory agencies for approval as an administrable protein therapeutic.

In the context of the present invention, described herein is a method of preparing a *Plasmodium* MSP-1 containing composition, said method comprising:
a) Expressing the fragment p83/30 of the gp190/MSP-1 protein from *Plasmodium* without heterologous sequences in a host cell;
b) Expressing the fragment p38/42 of the gp190/MSP-1 protein from *Plasmodium* without heterologous sequences in a host cell;
c) Recovering the fragment p83/30 and the fragment p38/42 from the host cells; and
d) Optionally combining the fragments p83/30 and p38/42 in a composition or as a combined preparation.

The expression of the fragments p83/30 and p38/42 may be carried out in a single host cell or separately in a first host cell and in a second host cell. The use of separate first and second host cells is preferred. The host cell may be provided by transfection with a nucleic acid encoding the respective MSP-1 fragment. Preferably, the nucleic acid encoding the p83/30 fragment and the nucleic acid encoding the p38/42 fragment may be located on an expression vector suitable for the respective host cell, e.g. a gram-negative bacterial cell such as an *E. coli* cell. The expression vector may be an episomal vector such as a plasm id, or a chromosomally integrated vector. The expression vector comprises the nucleic acid in operative linkage with a suitable expression control sequence, e.g. which may comprise a constitutive or an inducible promoter. The expression vector may comprise further genetic elements, e.g. an origin of replication, a selection marker, etc. Examples of suitable cloning vectors are disclosed in Sambrook et al., Molecular Cloning, A Laboratory Manual (1989), Cold Spring Harbor Laboratory Press, and other standard textbooks.

When the host cells are bacterial cells, e.g. *E. coli cells,* the fragments are preferably expressed in insoluble form, e.g. as inclusion bodies, which are discussed hereinabove. Preferably, the fragments are separately recovered as inclusion bodies which may be separately solubilized, e.g. in the presence of chaotropic salts such as guanidinium hydrochloride and subsequently refolded, e.g. in the presence of arginine and thiol reagents such as glutathione. Alternatively, the p83/30 and p38/42 fragments may be combined before solubilization, after solubilization or after refolding in a suitable molar ratio. The pulsed renaturation techniques discussed hereinabove may be used in this regard. After refolding, the fragments are transferred to a suitable buffer which may comprise a non-ionic surfactant such as Tween 80, Tween 20 or Triton X-100. If desired. The fragments may be separately purified by subsequent processing steps, comprising at least one of the following: filtration, anion and/or cation exchange chromatography such as Q-sepharose HP-chromatography, or SP-sepharose HP-chromatography, conditioning and concentration, e.g. by ultrafiltration.

According to step (d), the fragments are combined. Preferably, the fragments are combined in about equimolar amounts as indicated above. The amount of the respective fragment may be determined by quantitative SDS-PAGE performed under reducing conditions. It is especially preferred that the fragments are combined in the absence of heterologous polypeptides such as albumin.

After combining, the fragments may be further purified, e.g. by size exclusion chromatography, e.g. using Sephacryl S 300-HR/GE. Further treatments may comprise filtration, concentration and sterilization, e.g. by sterile filtration.

The nucleic acids encoding the fragments p83/30 and p38/42 may have reduced AT-content compared to the wild type sequence as described in WO 98/14583.

Generally, efficient production of the p38/42 fragment, e.g. having an amino acid sequence as set out in SEQ ID NO: 4 or 5 does not suffer from the same problems of low yield observed when producing the p83/30 fragment from unmodified nucleic acid. As used herein, "unmodified nucleic acid" denotes nucleic acid which, with respect to the coding sequence for the individual MSP-1 subunits p83 and p30 in a single polypeptide chain, corresponds to the native nucleic acid sequences (or native nucleic acid sequences which have been previously modified to increase stability as for example disclosed in WO 98/14583 and Pan et al. (1999) Nucleic Acids Res. 27(4), 1094-103) for the respective subunits in *Plasmodium,* preferably in *P. falciparum.* Generally, it should thus be possible to use a nucleic acid comprising native, unmodified sequences encoding the p38 and p42 subunits to efficiently produce the MSP-1 fragment p38/42. However, corresponding modification of the nucleic acid encoding the p38/42 fragment of MSP-1 may be carried out in a fashion analogous to that described above for the p83/30 fragment if desired, expedient or necessary. The advantages described above for the p83/30 fragment of MSP-1, for example efficient production in the form of inclusion bodies and omission of any heterologous sequences, e.g. isolation tags, thus also apply to the p38/42 subunit as well.

The inventors have determined that the two MSP-1 subunit fragments p83/30 and p38/42 spontaneously refold and associate under suitable conditions. This is especially the case when the fragments have previously been resolubilized out of inclusion body form. These conditions are described in detail herein. As such, it is possible to efficiently construct heterodimeric MSP-1 protein comprising the synthetic MSP-1 subunits p83/30 and p38/42 in a single concerted process without the need for prior yield-lowering refolding procedures for the individual subunits. The yield of the resulting MSP-1 protein is thus maximized. Further, since refolding and reassembly of MSP-1 take place spontaneously, traditional column-based protein isolation procedures may in most cases be dispensed with and, with them, the need for isolation tags of any sort, as described hereinabove. Of course, such column purification methods as described herein above may still be employed if desired.

As used herein, "mixing" or "combining" denotes bringing the p83/30 and p38/42 subunit fragments of MSP-1 into contact with one another under conditions which promote heterodimer formation. Suitable conditions to this end are described hereinbelow.

One very advantageous feature of this is that the two "halves" of MSP-1, i.e. the p83/30 and p38/42 fragments may be brought together as solubilized but non-refolded inclusion bodies to form complete MSP-1. Thus this embodiment avoids the need for separate refolding of the individual p83/30 and p38/42 subunits prior to mixing with one another. A solution containing both of the solubilized but still unfolded protein subunits may then be treated so that the individual subunits gradually renature or "refold" with one another, spontaneously reassembling the full, correctly folded MSP-1 heterodimer as they do. As indicated above, it is advantageous to combine the two subunits in stoichiometric, or near-stoichiometric amounts (i.e. in a ratio of 1:1 or close to 1:1). As such, the protein concentrations of the inclusion body preparations of the respective MSP-1 fragments should be determined in advance of mixing. The ability to reconstitute the MSP-1 heterodimer in one concerted step starting from still insoluble inclusion bodies thus eliminates any need to renature each of the subunits separately prior to mixing, a process which can incur product loss. As such, the possibility of mixing the individual protein subunits without any other prior treatment steps than recovery of inclusion bodies (and their optional prior solubilization) thus exploits the high protein yields obtained for the p83/30 subunit by appropriate nucleic acid modification and for the p38/42 by using the native or modified *Plasmodium* sequences to the maximum extent possible.

In one embodiment, the constituent p83/30 and p38/42 subunits of the MSP-1 heterodimer may be incorporated into the same or different cells, preferably prokaryotic cells. However, It is preferred in one embodiment that the combining step above is performed such that the p83/30 and p38/42 subunits are mixed in an approximate stoichiometric ratio of 1:1 in relation to one another. To this end, it is preferred that the p83/30 and p38/42 subunits of the MSP-1 heterodimer be expressed from separate cells. This enables a greater degree of experimental control in adjusting the stoichiometric ratio in which the protein subunits are later mixed such that this ratio is as close to 1:1 as possible.

In one embodiment, the individual subunit inclusion bodies are solubilized separately, i.e. in separate solutions prior to mixing for reassembly. One can then determine their respective amounts in solution, and combine appropriate amounts of each solution to reconstitute an entire MSP-1. In one preferred embodiment, the two solutions containing the p83/30 and p38/42 subunits can be added as metered aliquots, i.e. "pulses" into one mixture solution, as discussed above. In one particularly preferred embodiment, the inventors have found that the reconstitution, i.e. reassembly of the MSP-1 heterodimer from its respective p83/30 and p38/42 halves may be promoted by timing the respective additions in the approximate middle of stirring periods lasting about 2 minutes. That is to say, following a respective incubation during which protein renaturation has been allowed to take place, the solution is gently stirred for about 1 minute. Stirring continues during protein addition, and stirring is continued for about one further minute. After this period, stirring is stopped and the solution is allowed to incubate at rest for a predetermined time period, advantageously. Following this rest period, the process is repeated until all available protein has been added.

In another embodiment, the p83/30 and p38/42 subunits are not renatured prior to the mixing step, but rather are added to one another in denatured form, as already described above. For example, the denatured form may include inclusion bodies which have been recovered from a cell lysate by centrifugation and resuspended (but not renatured) prior to the mixing step. In one embodiment, the mixture solution containing denatured p83/30 and p38/42 is subjected to the same type of pulse treatment as described above.

In another embodiment, one of the two protein subunits of the MSP-1 heterodimer is refolded prior to being mixed with the other of the two subunits, the latter being still denatured and soluble or insoluble upon mixing. Here, as above, a pulsing process may be employed to solubilize the as yet unsoluble subunit (if needed), and simultaneously promote association of the two protein subunits into correctly folded MSP-1.

In one embodiment, actual recovery of the MSP-1 heterodimer can be accomplished for example by diafiltration or dialysis with salt exchange to any buffer desired without the need of including stabilizing components such as BSA or HSA. A range of different buffers may be used, as required for subsequent purification and formulation processes.
A further aspect of the invention provides the modified nucleic acid as set out above, or the vector as set out above for use as a vaccine.

As mentioned above, the fully reconstituted MSP-1 heterodimer as well as the individual p83/30 and p38/42 protein subunits thereof are capable of eliciting an immune response when brought into contact with the immune system of a host, i.e. the immune system of an animal or a human. Especially preferred to this end is the use of the MSP-1 heterodimer comprising both the p83/30 and p38/42 subunits, as the heterodimer will present the host immune system with epitopes most closely matching those of the native MSP-1 heterotetramer. However, also preferred is the use of either of the protein subunits p83/30 or p38/42 to this end, as these will present the host immune system with certain epitopes equivalent to those in heterodimeric MSP-1.

In one embodiment, the vaccine comprises a pharmacologically compatible carrier, i.e. adjuvant Pharmacologically compatible adjuvants in this respect are all carriers and agents known in the art to enhance the immune response. Preferred adjuvants in this regard include aluminium oxide and aluminum phosphate carriers, each with or without additional stimulating compounds such as MPL, ISA 720 and ISA 51 from Seppich SA, IC31 from Intercell or MF59.

In one embodiment, the vaccine is suitable for administration by injection, for example intradermal, intramuscular or subcutaneous. In general, however, any route of administration known to lead to an immune response in vaccine administration may be used to administer the inventive vaccine.

The vaccine may be prepared as a reconstitutible lyophilisate, as a liquid, e.g. as a solution or a suspension, or as an emulsion, e.g. as a water-in-oil emulsion. A preferred dosage of the vaccine comprises 1-500 µg, more preferably 20-100 µg for application in human medicine. The vaccine may be administered in a single dose or in multiple, doses. The administration in multiple doses is preferred. Doses of 100 µg are especially preferred.
A further aspect of the invention provides a use of the modified nucleic acid as set out above, or the vector as set out above, in the manufacture of a medicament for preventing and/or treating malaria.

In one embodiment, the medicament is prepared for administration together with an adjuvant, wherein exemplary adjuvants are as set out above. Exemplary routes and amounts of administration are also as set out above.

In a preferred embodiment, the medicament is prepared for administration together with ISA720. The inventors have surprisingly found that the administration of MSP-1 comprising the protein subunits p83/30 and p38/42 (each without heterologous sequences) to animals leads to an unusually high antibody titer, as evidenced by a) comparison with titers obtained using Freund's complete/incomplete adjuvant, the most potent adjuvant known, and b) examination of the specificity of the antibodies obtained (high titers of antibodies were obtained also against the low immunogenicity regions p30 and p38). Thus, the modifications made in the p83/30-encoding nucleic acid not only allow production of the corresponding subunit for the first time in acceptable yield and free of any isolation tags, but also the finding of a beneficial antigen/adjuvant combination resulting in a promising formulation of MSP-1 from *Plasmodium,* together with the adjuvant ISA720.

The embodiments above in the context of pharmaceutically compatible carriers/adjuvants/diluents as well as administration routes and amounts apply to this aspect of the invention *mutatis mutandi.*
A further aspect of the invention provides a vaccine comprising the modified nucleic acid as set out above, or the vector as set out above .

In the context of the present invention, described herein is a composition comprising a purified fragment p83/30 of the gp190/MSP-1 protein from a *Plasmodium* F-strain. The p83/30 fragment preferably comprises the sequence as shown in SEQ ID NO: 3 or a modified sequence as described above. This composition is preferably used as a pharmaceutical preparation, e.g. as a vaccine as described above. With regard to the preferred characteristics, e.g. purity and/or content of degradation products, of this composition, reference is made to the disclosure as described above.

The embodiments above in the context of pharmaceutically compatible carriers/adjuvants/diluents as well as administration routes and amounts apply to this aspect *mutatis mutandi.*
The invention will now be illustrated by way of the following non-limiting examples.

### Examples

### Example 1: Mutation of nucleic acid sequences encoding MSP-1 protein subunits

In prokaryotes the ribosome binding site, also referred to as "Shine-Dalgamo-sequence", consists of purine-rich sequences approximately 10 nucleotides upstream of the start codon. The ribosomal binding site sequence in the plasmid pZE23/d-83/30 used for the expression of the recombinant MSP-1 proteins corresponds to the "Shine-Dalgano" consensus sequence (Shine & Dalgarno (1975) Nature 254, 34-8) and its capacity of driving gene expression in E. *coli* had been described previously (Lutz & Bujard (1997) Nucleic Acids Res. 25, 1203-10). Due to the fact that in prokaryotes translation of mRNA into protein takes place while transcription is still ongoing, the secondary structure of the mRNA might influence the recognition of the mRNA by ribosomes and, as a consequence, the rate of protein synthesis.

In light of the above, *in silico* analysis of the mRNA structure was performed by submitting about the first 150-200 bp of the mRNA encoding p83/30 (i.e. the first 150-200 bp of either of SEQ ID NO: 6 or 7) to the RNA structure prediction program "mfold" (version 3.2) provided as an online resource by Rensselaer Polytechnic Institute, Troy, NY (Zuker (2003) Nucleic Acids Res. 31 (13), 3406-15; http://www.bioinfo.rpi.edu/applications/). Default values were used for the prediction of RNA secondary structure. These default values are designed to reflect conditions *in vivo.* In contrast to parameters for the prediction of secondary structure in DNA, parameters for RNA folding cannot be adjusted with the user interface of the "mfold" server. SEQ ID NO: 6 and 7 are shown in Figs. 15 and 16, respectively.

The predicted structure of the ribosomal binding site was found to contain a hairpin-like structure, which might restrict access of ribosome (see Fig.1). It was hypothesized that by modifying the secondary structure of the mRNA such that the ribosomal binding site is no longer part of a structurally well-defined region, such as the hairpin-like structure, ribosomal access to the mRNA might be facilitated, thus promoting synthesis of recombinant MSP-1 in the bacteria.

To this end, several mutations were introduced into sequences interacting with the ribosome in order to destabilize the hairpin-like structure. The modified sequences were again subjected to computational analysis and showed the expected effect of the mutations, i.e. the structure of the ribosomal binding site was no longer part of a hairpin-like structure.

Following this computational prediction, two different sets of the computed mutations were introduced by site-specific mutagenesis into the p83/30-encoding nucleic acids. Sequences with either set of mutations as well as a sequence containing both sets of mutations were finally cloned and all lead to improved MSP-1 expression as compared to the original sequence. The sequence containing both sets of mutations proved to be most effective and was then used for the expression of p83/30. Both sets of mutations are present in SEQ ID NO: 8 and 9 in identical form (see Figs 17 and 18). These mutations relative to the parental nucleic acid sequences of SEQ ID NO: 6 and 7, respectively, are silent mutations, meaning that they do not lead to modifications at the amino acid level. The nucleotides in SEQ ID NO: 8 and 9 which were mutated relative to SEQ ID NO: 6 and 7 are emphasized in respective SEQ ID NO: 8 and 9 in lower case boldface, and the start codon ATG is underlined (see Figs. 17 and 18).

### Example 2: Comparison of expression of non-mutated with mutated MSP-1 subunits.

*E. coli* W3110-Z2 containing pZE23/d-83/30 (unmodified sequence for the p83/30 fragment, corresponding to SEQ ID NO: 6 or 7, in which the ribosomal binding sites are identical) or pZE23/d-mut83/30 (modified sequence for the p83/30 fragment, corresponding to SEQ ID NO: 8 or 9, in which the ribosomal binding sites have been identically modified) were grown in Superbroth medium at 37°C to a cell density of OD500 = 0.5. MSP-1 expression was induced by addition of IPTG (1 mM) and bacteria were grown for an additional 4 hours. Aliquots of whole bacterial extracts were taken at the time of induction (0 h) and after 2 (2h) and 4 hours (4h), respectively. Whole cell extracts were analyzed by SDS-PAGE and stained with Coomassie. The results obtained are shown in Fig. 2.

### Example 3: Production of heterodimeric MSP-1 from p83/30 and p38/42

### Example 3a: Production of host cells with translationally optimized p83/30 and p38/42

CaCl₂-competent *E. coli* W3110Z2 are transformed with plasmids pZE23/mut-d-83/30 and pZE23/d38/42. *E.coli* host strains W3110Z2-pZE23/d83/30 and W3110Z2-pZE23/d38/42 are grown separately, but under identical conditions: in Superbroth at 37°C. At cell densities of OD600 = 7, synthesis of the MSP-1 subunits is induced by addition of Isopropyl beta-D-1-thiogalactopyranoside ("IPTG"; final concentration 1 mM). At OD = 18-24 cells are harvested by centrifugation and either stored at - 80°C or immediately processed further. The results are shown in Fig. 3 for the expression of p83/30 and p38/42 fragments from mutated and non-mutated nucleic acid sequences, respectively.

### Example 3b: Expression of p83/30 and p38/42 subunits

Fermentations of the two *E. coli* host strains W3110Z2-pZE23/d83/30 and W3110Z2-pZE23/d38/42 were carried out separately, but under identical conditions: in Superbroth at 37°C. At cell densities of OD600 = 7 synthesis of the MSP-1 fragments was induced by addition of IPTG (final concentration 1 mM). At OD = 18-24 cells are harvested by centrifugation and either stored at - 80°C or immediately processed further. The results of the fermentation are shown in Fig. 4.

### Example 3c: Recovery and solubilization of p83/30 and p38/42 subunits from inclusion bodies

Cells from fermetation or from frozen biomass were suspended in lysis buffer at 4°C and opened via a fluidizer at 1.034x10⁵ - 1.379x10⁵ kPa (15,000 - 20,000 psi). Centrifugation at 27,000 g was performed for 20 min. The pellet contained the inclusion bodies. The pellet was resuspended in lysis buffer (PBS, pH 7.4, lysozyme, Dnase), and centrifugation was repeated. The pellet was harvested and subjected to another cycle of resuspension and centrifugation. The final concentration of inclusion bodies was 500 mg/L bacterial culture. The inclusion bodies collected in the pellet were dissolved in solubilization buffer (6 M guanidinium hydrochloride, 50 mM sodium phosphate, pH 8.0, 10 mM DTT, 1mM EDTA) while stirring at room temperature for 10 hours. Centrifugation at 27,000 g for 40 min cleared the solution of dissolved inclusion bodies. Fig. 5 shows electrophoretic analysis of solubilized inclusion bodies.

### Example 3d: Mixing and renaturation of p83/30 and p38/42 to form reassembled MSP-1 heterodimer

The content of the MSP-1 fragments p83/30 and p38/42 in their respective solubilized inclusion body preparations was determined by electrophoretic analysis of a dilution series of both preparations. These determinations are important for calculating the volumes of the two preparations to be combined during the pulse renaturing process. Both subunits were added to the renaturation buffer (500 mM arginine, 50 mM sodium phosphate, pH 8.0, 1 mM EDTA, 0.1 mM glutathione) in ten equal aliquots, such that the final concentration was 0.5 mg/mL renatured heterodimer at the end of the renaturation process.

The individual aliquots were added to the renaturing buffer in the following way: The solution was stirred for 1 min before, during and after each addition, i.e. "pulse" of protein. About 1 hour was allowed to elapse between stopping stirring following one addition and starting stirring prior to the next respective addition. Following the last addition, the solution was incubated for an additional 10 hrs. The entire process was temperature controlled at 10°C. Following incubation, the protein solution was centrifuged or diafiltered at 4°C. The buffer was exchanged by diafiltration. Protein recovery was 99%.

### Example 3e: Purification of the reconstituted MSP-1 heterodimeric protein

The renatured MSP-1 heterodimer comprising both the p83/30 and p38/42 fragments without heterologous polypeptide sequences such as isolation tags was subjected to three chromatographic fractionation processes: HP-Q sepharose chromatography (AP Biotech), hydroxyapatite chromatograohy, macroprep Type I (BioRad), and HP-SP sepharose chromatography (AP Biotech).

The overall yield and purity of the MSP-1 Heterodimer per 100 L fermentation volume was 8 gm of MSP-1. This is an amount of MSP-1 sufficient for 80,000 doses of 100 µg each. The end product contained less than 1 ng of *E*. *coli* protein per 1 mg of MSP-1.

About 85% of the final material consists of intact MSP-1 heterodimeric protein. The end-product contained about 15% of so-called product-derived impurities. These impurities are the product sensitivity to protease. Of the two fragments p83/30 and p38/42, it was primarily the p83/30 subunit which showed such sensitivity to protease. The degradation products (see Fig. 6) appear to constitute parts of incomplete heterodimers, as they cannot be separated from the complex. All detectable components of the purified MSP-1 preparation can be identified as degradation products of MSP-1 (Fig. 6), as determined using appropriate antibodies specific for the p83/30 and p38/42 fragments of MSP-1.

### Example 4: Immunogenicty studies

Immunogenicity of an antigen strongly depends on (1) the way it is formulated, i.e. with which adjuvant it is combined; (2) the immunization regimen, i.e. the way it is administered (for example, subcutaneously, s.c. or intramuscularly, i.m.); (3) the number of immunizations carried out; and (4) the temporal sequence of the immunizations.

In the present study, examination of adjuvants was limited to those which are suitable for use in humans. Freud's complete and incomplete adjuvant (FCA, FIA), a very potent adjuvant not accepted for use in humans, was used as a positive control. A further control was pure antigen in phosphate buffered saline (PBS).

A first set of immunizations was carried out in a mouse model. Antigen/adjuvant combinations which showed a high immunogenicity in these animals as verified by high titers of MSP-1-specific antibodies were subsequently examined in rabbits.

### Example 4a: Immunization in Balb c mice

Groups of seven animals were injected with the antigen/adjuvant combination containing 5 µg of antigen per dose. Three subcutaneous injections were performed on days 0, 21 and 42. On day 63, animals were bled and final serum was harvested. Antibody titers were determined via ELISA using MSP-1 heterodimer as capture protein.

Immunizations were carried out with the following adjuvants: aluminum hydroxide; aluminum phosphate; MPL (Sigma); CpG (Qiagen); IC31 (Intercell, Vienna, Austria); Montanide ISA 720 (Seppich, Paris, France); Montanide 51 (Seppich); Virosomes (Bema Biotech, Bem, Switzerland); and FCA/FIA.

Results of this study are shown in Fig.7. The identities of the individual clusters of the data bars A-Y are as set out in the corresponding description of Fig. 7 hereinabove. Importantly, data cluster A represents antigen administered in PBS, data cluster X, Y represents antigen administered in FCA/FIA. As can be seen in Fig.7, data clusters K (corresponding to the adjuvant X3), S (corresponding to the adjuvant Montanide ISA720) and T (corresponding to the adjuvant Montanide ISA51) resulted in the highest antibody titers of all test formulations. Based on these results, MSP-1 and MSP-1-related antigen was subsequently administered with each of these three adjuvants to rabbits.

### Example 4b: Immunization of rabbits

Groups of five rabbits were injected intramuscularly with four doses of formulated antigen (100 µg per dose). Immunizations were performed on days 0, 28, 42 and 56. On day 70 animals were bled and sera were harvested. The titer of MSP-1-specific antibodies was measured via ELISA using MSP-1 heterodimer as well as the 4 subunits p83, p30, p38, and p42 as capture proteins. Results are shown in Fig. 8.

As Figs. 7 and 8 each show, there exist several promising antigen/adjuvant combinations and further studies are likely to reveal additional candidates. Present data indicate, however, that the combination of MSP-1 heterodimer with Montanide ISA 720 is particularly promising, for two surprising reasons:
(1) The antibody titer obtained in rabbits surpasses the combination of MSP-1 with Freund's adjuvant.
(2) The titer of antibodies when analyzed using the four subunits of MSP-1 show a most interesting profile. Titers against p30 and p38 are unusually high in comparison to those specific for p83 and p42. As the two subunits p30 and p38 elicit antibodies which are particularly effective in inhibiting parasite growth, the outcome of this study is particularly promising.

## Claims

1. A method for preparing a modified nucleic acid encoding the p83/30 fragment of the merozoite surface protein 1 (MSP-1) from *Plasmodium* having an amino acid sequence as set out in SEQ ID NO: 1, 2 or 3, said method comprising:
a) Providing an mRNA sequence encoding the p83/30 fragment of the MSP-1 from *Plasmodium* having an amino acid sequence as set out in SEQ ID NO: 1, 2 or 3;
b) Subjecting the region surrounding the start codon of the mRNA sequence of step (a) to analysis *in silico* to determine the predicted secondary structure of said mRNA sequence;
c) Identifying at least one stable, hairpin-like structure in said region surrounding the start codon of step (b);
d) Modifying the sequence of the mRNA in said region surrounding the start codon of step (b), wherein at least one nucleotide substitution, insertion, deletion and/or inversion destabilizes said hairpin-like region, and wherein the at least one nucleotide substitution, insertion, deletion and/or inversion is effected *in silico* so as to encode the same amino acid sequence as encoded by said unmodified nucleic acid sequence;
e) Subjecting the region of step (d) to analysis *in silico* under the same conditions as set out in step (b);
f) Determining whether the at least one nucleotide substitution, insertion, deletion and/or inversion destabilizes the at least one hairpin-like region containing the at least one ribosomal binding site;
g) Optionally repeating steps (d) - (f) as needed until analysis *in silico* under the same conditions as set out in step (b) indicates the destabilization of the at least one hairpin-like region;
h) Preparing the corresponding modified nucleic acid.

2. A modified nucleic acid encoding the p83/30 fragment of the merozoite surface protein 1 (MSP-1) of *Plasmodium* having an amino acid sequence as set out in SEQ ID NO: 1, 2 or 3, wherein the nucleic acid comprises a nucleotide sequence as set out in any of SEQ ID NOs: 8 or 9.

3. A vector comprising the modified nucleic acid of claim 2.

4. The vector of claim 3, wherein the vector is a polynucleotide vector or a viral vector.

5. A method of preparing the p83/30 fragment of the merozoite surface protein 1 (MSP-1) from *Plasmodium,* said method comprising:
a) Providing a modified nucleic acid of claim 2 and/or a vector of claim 3 or 4;
or
preparing a modified nucleic acid by the method of claim 1;
b) Incorporating the modified nucleic acid and/or vector of step (a) into a cell, preferably a prokaryotic cell;
c) Incubating the cell of step (b) under conditions allowing expression of the p83/30 fragment of the MSP-1; and
d) Recovering the p83/30 fragment of the MSP-1.

6. The method of claim 5, wherein said recovering step comprises solubilizing said p83/30 fragment of the MSP-1 from inclusion bodies.

7. Use of the modified nucleic acid of claim 2 or the vector of claim 3 or 4 in the manufacture of a medicament for preventing and/or treating malaria.

8. The use of claim 7, wherein the medicament is prepared for administration together with an adjuvant chosen from the group consisting of: aluminium hydroxide/MPL, aluminium phosphate/MPL, ASO2, ISA51, IC31, alum, ISA720 and/or MF59.

9. A vaccine comprising the modified nucleic acid of claim 2 or the vector of claim 3 or 4.

## Patentansprüche

1. Verfahren zum Herstellen einer modifizierten Nukleinsäure, welche das p83/30-Fragment des Merozoid-Oberflächenproteins 1 (merozoite surface protein 1, MSP-1) von *Plasmodium* mit einer Aminosäuresequenz wie den SEQ ID Nr.: 1, 2 oder 3 dargestellt, kodiert, wobei das Verfahren umfaßt:
a) Bereitstellen einer mRNA Sequenz, welche das p83/30-Fragment von MSP-1 von *Plasmodium* mit einer Aminosäuresequenz wie in SEQ ID Nr.: 1,2 oder 3, dargestellt, kodiert;
b) Unterwerfen der Region, welche das Start-Codon der mRNA Sequenz von Schritt (a) umgibt, einer *in silico* Analyse, um die vorhergesagte Sekundärstruktur der mRNA-Sequenz festzustellen;
c) Identifizieren von mindestens einer stabilen Haarnadel-ähnlichen Struktur in der Region, welche das Start-Codon von Schritt (b) umgibt;
d) Modifizieren der Sequenz der mRNA in dem Bereich, welche das Start-Codon von Schritt (b) umgibt, wobei mindestens eine Nukleotid-Substitution, -Insertion, -Deletion und/oder -Inversion die Haarnadel-ähnliche Region destabilisiert und wobei die mindestens eine Nukleotid-Substitution, -Insertion, -Deletion und/oder -Inversion *in silico* so ausgeführt wird, daß sie dieselbe Aminosäuresequenz kodiert, die durch die unmodifizierte Nukleinsäuresequenz kodiert wird;
e) Unterwerfen des Bereichs nach Schritt (d) einer *in silico* Analyse unter den gleichen Bedingungen wie in Schritt (b) dargelegt;
f) Bestimmen, ob die mindestens eine Nukleotid-Substitution, -Insertion, -Deletion und/oder -Inversion die mindestens eine Haarnadel-ähnliche Region, welche mindestens eine ribosomale Bindungsstelle enthält, destabilisiert;
g) optional Wiederholen der Schritte (d) - (f) nach Bedarf bis die *in silico* Analyse unter den gleichen Bedingungen wie in Schritt (b) dargelegt, die Destabilisierung der mindestens einen Haarnadel-ähnlichen Region anzeigt;
h) Herstellen der entsprechenden modifizierten Nukleinsäure.

2. Modifizierte Nukleinsäure, welche das p83/30-Fragment des Merozoit-Oberflächenproteins 1 (merozoite surface) Protein 1 (MSP-1) von *Plasmodium* mit einer Aminosäuresequenz wie in SEQ ID Nr.: 1, 2 oder 3 dargelegt, kodiert, wobei die Nukleinsäure eine Nukleotidsequenz, wie in SEQ ID Nr.: 8 oder 9 dargelegt, umfaßt.

3. Vektor umfassend die modifizierte Nukleinsäure nach Anspruch 2.

4. Vektor nach Anspruch 3, wobei der Vektor ein Proteinnukleotidvektor oder ein viraler Vektor ist.

5. Verfahren zum Herstellen eines p83/30 Fragments von Merozoid-Oberflächenprotein 1 (merozoite surface protein 1), (MSP-1) von *Plasmodium,* wobei das Verfahren umfaßt:
a) Bereitstellen einer modifizierten Nukleinsäure nach Anspruch 2 und/oder eines Vektors nach Anspruch 3 oder 4;
oder
Herstellen einer modifizierten Nukleinsäure durch das Verfahren nach Anspruch 1;
b) Einbringen der modifizierten Nukleinsäure und/oder des Vektors nach Schritt (a) in eine Zelle, vorzugsweise einer prokaryotische Zelle;
c) Inkubieren der Zelle nach Schritt (b) unter Bedingungen, welche die Expression des p83/30-Fragments von MSP-1 erlauben; und
d) Gewinnen des p83/30-Fragments von MSP-1.

6. Verfahren nach Anspruch 5, wobei der Schritt des Gewinnens ein Solubilisieren des p83/30-Fragments von dem MSP-1 aus Einschlußkörpern umfaßt.

7. Verwendung der modifizierten Nukleinsäure nach Anspruch 2 oder des Vektors nach Anspruch 3 oder 4 in der Herstellung eines Medikaments zum Verhindern und/oder Behandeln von Malaria.

8. Verwendung nach Anspruch 7, wobei das Medikament präpariert ist für die Verabreichung zusammen mit einem Adjuvans, ausgewählt aus der Gruppe bestehend aus: Aluminiumhydroxid/MPL, Aluminiumphosphat/MPL, ASO2, ISA51, IC31, Alaun, ISA720 und/oder MF59.

9. Impfstoff umfassend die modifizierte Nukleinsäure nach Anspruch 2 oder den Vektor nach Anspruch 3 oder 4.

## Revendications

1. Procédé de préparation d'un acide nucléique modifié, codant le fragment p83/30 de la protéine de surface-1 du mérozoïte (MSP-1) de *Plasmodium,* ayant une séquence d'acides aminés donnée en SEQ ID N°1, 2 ou 3, ledit procédé comprenant :
a) se procurer une séquence d'ARNm codant le fragment p83/30 de MSP-1 de *Plasmodium,* ayant une séquence d'acides aminés donnée en SEQ ID N°1, 2 ou 3 ;
b) soumettre la région entourant le codon d'initiation de la séquence d'ARNm de l'étape (a) à une analyse *in silico* pour déterminer la structure secondaire prédite de ladite séquence d'ARNm ;
c) identifier au moins une structure stable de type en épingle à cheveux dans ladite région entourant le codon d'initiation de l'étape (b) ;
d) modifier la séquence de l'ARNm dans ladite région entourant le codon d'initiation de l'étape (b), où au moins une substitution, insertion, délétion et/ou inversion de nucléotide déstabilise ladite région de type en épingle à cheveux, et où la au moins une substitution, insertion, délétion et/ou inversion de nucléotide est effectuée *in silico* de manière à coder la même séquence d'acides aminés que celle codée par ladite séquence d'acide nucléique non modifiée ;
e) soumettre la région de l'étape (d) à une analyse *in silico* dans les mêmes conditions que celles de l'étape (b) ;
f) déterminer si la au moins une substitution, insertion, délétion et/ou inversion de nucléotide déstabilise la au moins une région de type en épingle à cheveux contenant le au moins un site de fixation du ribosome ;
g) éventuellement, répéter les étapes (d) - (f) si nécessaire, jusqu'à ce que l'analyse *in silico* dans les mêmes conditions que celles de l'étape (b), montre une déstabilisation de la au moins une région en tête d'épingle ;
h) préparer l'acide nucléique modifié correspondant.

2. Acide nucléique modifié codant le fragment p83/30 de la protéine de surface-1 du mérozoïte (MSP-1) de *Plasmodium,* ayant une séquence d'acides aminés donnée en SEQ ID N°1, 2 ou 3, où l'acide nucléique comprend une séquence de nucléotides telle que donnée en SEQ ID N°8 ou 9.

3. Vecteur comprenant l'acide nucléique modifié de la revendication 2.

4. Vecteur selon la revendication 3, où le vecteur est un vecteur polynucléotidique ou un vecteur viral.

5. Procédé de préparation du fragment p83/30 de la protéine de surface-1 du mérozoïte (MSP-1) de *Plasmodium,* le procédé comprenant:
a) se procurer un acide nucléique modifié selon la revendication 2 et/ou un vecteur selon la revendication 3 ou 4 ; ou
préparer un acide nucléique modifié par le procédé de la revendication 1 ;
b) incorporer l'acide nucléique modifié et/ou le vecteur de l'étape (a) dans une cellule, de préférence une cellule procaryote ;
c) incuber la cellule de l'étape (b) dans des conditions permettant l'expression du fragment p83/30 de MSP-1, et
d) récupérer le fragment p83/30 de MSP-1.

6. Procédé selon la revendication 5, où ladite étape de récupération comprend la solubilisation dudit fragment p83/30 de MSP-1 à partir de corps d'inclusion.

7. Utilisation de l'acide nucléique modifié de la revendication 2 ou du vecteur de la revendication 3 ou 4 dans la préparation d'un médicament destiné à prévenir et/ou traiter la malaria.

8. Utilisation selon la revendication 7, où le médicament est préparé pour une administration avec un adjuvant choisi parmi le groupe consistant en : hydroxyde d'aluminium/MPL, phosphate d'aluminium/MPL, ASO2, ISA51, IC31, alun, ISA720 et/ou MF59.

9. Vaccin comprenant l'acide nucléique modifié de la revendication 2 ou le vecteur de la revendication 3 ou 4.
